# EUROPEAN PATENT APPLICATION

(11) **EP 3 590 508 A1**
(43) Date of publication of application: **08.01.2020**
(21) Application number: 18761258.5
(22) Date of filing: 02.03.2018
(51) Int. Cl.: A61K 9/50, A61K 9/127, A61K 9/107, A61K 39/00

(54) **MULTI-DOMAIN VESICLE COMPRISING IMMUNOACTIVE MATERIAL, PRODUCTION METHOD THEREFOR AND IMMUNOMODULATORY COMPOSITION COMPRISING SAME**

(30) Priority: 02.03.2017 KR 20170027303; 28.02.2018 KR 20180024900
(71) Applicant: Dandi Bioscience Inc, Seoul 05029 (KR)
(72) Inventor: LIM, Yong Taik, Seongnam-si Gyeonggi-do 13599 (KR)
(74) Representative: Dehns
(86) International application number: PCT/KR2018/002516
(87) International publication number: WO 2018/160026

(57) **Abstract**

The present invention relates to a multi-domain vesicle comprising an immunostimulatory material, a production method of the multi-domain vesicle and an immunomodulatory composition comprising the multi-domain vesicle. According to one aspect of the present invention, the multi-domain vesicle comprises: at least two liposomes making contact and connected with each other, and a multi-domain vesicle outer wall surrounding the at least two liposomes. The multi-domain vesicle is formed from an oil phase and an aqueous phase, wherein the oil phase comprises a first immunomodulatory material and a fluid oil; the oil phase forms a membrane of the liposomes, and the multi-domain vesicle outer wall; the aqueous phase comprises a second immunomodulatory material; the aqueous phase is an internal aqueous phase of the membrane of the liposomes, and an outer aqueous phase of the membrane of the liposomes; the first immunomodulatory material is a fat-soluble immunostimulatory material; the second immunomodulatory material is a water-soluble immunostimulatory material; and the fluid oil improves the structural stability of the at least two liposomes making contact and connected with each other.

## Description

### [Technical Field]

The present invention relates to multi-domain vesicle comprising immunostimulatory material, production method of the multi-domain vesicle and immunomodulatory composition comprising the multi-domain vesicle.

### [Background Art]

Currently, liposomal materials encapsulating various drugs are being used. However, in the technique using such a single liposomal material, low loading efficiency and in vivo instability are pointed out as major disadvantages.

Recently, in order to activate immune cells, various liposomes and emulsion materials loaded with immunostimulatory materials (for example, ASO1, ASO2, and AS15 from GSK and MF59 from Novartis AG) have been used as immunostimulatory materials for preventing or treating various infectious diseases and cancers. The single liposome-based materials are vaccine compositions for preventing infectious diseases, and are currently at the clinical trial stage, but due to the low duration time of antigens and immunostimulatory materials, there was a disadvantage in that such a material had to be additionally injected two to three times at regular intervals.

In order to overcome the disadvantages, the Darrell Irvine group at MIT recently developed an immunostimulatory cancer vaccine with a multilamellar liposome structure (Nature Materials, 10, 243-251, 2011). The cancer vaccine was an attempt to solve the low encapsulation efficiency and stability problems, which were the fundamental disadvantages of a single liposomal material by loading an antigen and immunostimulatory materials inside a liposome with a multilamellar structure, and then using multivalent metal ions or a chemical linker in each lipid layer to create a chemical crosslinking structure.

However, since the form of the liposome with the multilamellar structure is very heterogeneous and the production process for producing a multilamellar structure with a specific structure is arbitrary, during overall production, there are disadvantages in that a vaccine composition having uniform characteristics cannot be obtained and since chemical crosslinking bonds are used, there is a limitation in that toxicity may be caused to the human body.

Further, as a similar form, a drug carrier called a multivesicular liposome in the related art has been disclosed by Kim Shin-Il's research team at the University of California [Biochimica Biophysica Acta 1983 Mar. 9 728 (3) 339-348], Mantripragada's research team in 2002 [Progress of Lipids Research 41 (2002) 392-406], Wafa's research team in 2007 [International Journal of Pharmaceutics 331 (2007) 182-185], and the like. The multivesicular liposome consists of a mixture of materials selected from the group consisting of neutral lipids, cholesterol and triolein.

In the multivesicular liposome in the related art, the principle that microvesicles maintain a cluster of microvesicles is that a triolein material between lipid membranes of individual liposomes fixes a double membrane so as not to be destroyed and scattered even in a rapid change in the curve of the lipid membrane to be contacted. These multivesicular liposomes are currently developed as a drug loaded with bupivacaine which is a pain management agent, and are commercially available under the trade name EXPAREL®.

However, the thus-prepared multivesicular liposomes have very low structure stabilization efficiency, so that there is a problem in that during the preparation process (for example, centrifugation, temperature change, and the like), microclusters disintegrate, resulting in a non-uniform size or shape. In addition, it has been investigated that no multivesicular liposomal form into which an immunostimulatory drug has been introduced has been found to date. Meanwhile, it is important to develop a technique capable of regulating immunosuppression in vivo in the regulation of immune function along with the immunostimulation technique. In particular, in order to solve the low therapeutic efficiency and side effects of anti-cancer immunotherapy, there is a very urgent need for developing a technique capable of overcoming an immunosuppression phenomenon in the cancer microenvironment.

Anti-cancer immunotherapy methods for treating cancer using an in vivo immune system have an advantage in which side effects may be minimized as compared to existing chemotherapy or radiotherapy methods. Among these anti-cancer immunotherapy techniques, a cell therapeutic agent method of activating therapeutic immune cells such as T cells (including CAR-T), dendritic cells, and natural killer cells in vitro, and then directly injecting the therapeutic immune cells into the body, an anti-cancer vaccine method of enhancing anti-cancer efficacy by injecting a cancer antigen and immunostimulatory materials into the body to directly activate immune cells present in the body, and the like have been actively studied. However, these cell therapeutic agents or anti-cancer vaccines are usually used for blood cancer-related diseases, and have a disadvantage in that most of the cell therapeutic agents or anti-cancer vaccines have a very low therapeutic efficacy against solid cancers.

One of these reasons is due to microenvironmental factors that suppress immune function around solid cancer. In fact, cells (myeloid-derived stromal cells (MDSCs), regulatory T cells (Treg), and tumor-associated macrophages (TAM)) reducing the function of immune cells, or cytokines causing immunosuppression, metabolites, and the like actively act in the tumor microenvironment, thereby rapidly reducing the activities of immunostimulatory materials and therapeutic immune cells. Accordingly, there is a very urgent need for developing a new therapeutic platform technique capable of controlling an immunosuppressive factor in a solid cancer microenvironment in order to increase the therapeutic efficiency against solid cancer.

Recently, studies have been actively conducted worldwide on the development of a drug capable of controlling various immunosuppressive factors in a tumor microenvironment. However, these drugs are easily degraded by various in vivo physiological environments and enzymes when injected into the body, or delivered to tissues other than a tumor site, and thus have a disadvantage in that various undesirable side effects are caused.

In order to overcome the disadvantages, in the actual clinical field, attempts have been made to enhance the immunotherapeutic effect by repeatedly administering a drug at high dose, but various drug toxicities and side effects have resulted in reducing therapeutic effects.

Therefore, there is a very urgent need for developing an anti-cancer immunotherapeutic agent capable of effectively targeting an immunosuppressive factor and minimizing side effects caused by drugs by releasing a drug capable of controlling immunosuppressive environmental factors which inhibit the therapeutic function of an immunotherapeutic agent around solid cancer by sustained release in a solid cancer microenvironment, and a technique for improving the therapeutic effect of an anti-cancer therapy using the same.

### [Disclosure]

### [Technical Problem]

The present invention provides a multi-domain vesicle comprising an immunostimulatory material, a production method of the multi-domain vesicle and an immunomodulatory composition comprising the multi-domain vesicle.

However, technical problems to be solved by the present application are not limited to the aforementioned problems, and other problems that are not mentioned may be clearly understood by those skilled in the art from the following description.

### [Technical Solution]

According to one aspect of the present invention, it is possible to provide a multi-domain vesicle comprising: at least two liposomes making contact and connected with each other, and a multi-domain vesicle outer wall surrounding the at least two liposomes. The multi-domain vesicle is formed from an oil phase and an aqueous phase, wherein: the oil phase comprises a first immunomodulatory material and a fluid oil; the oil phase forms a membrane of the liposomes, and the multi-domain vesicle outer wall; the aqueous phase comprises a second immunomodulatory material; the aqueous phase is an internal aqueous phase of the membrane of the liposomes, and an outer aqueous phase of the membrane of the liposomes; the first immunomodulatory material is a fat-soluble immunostimulatory material; the second immunomodulatory material is a water-soluble immunostimulatory material; and the fluid oil improves the structural stability of the at least two liposomes making contact and connected with each other.

According to another aspect of the present invention, it is possible to provide an immunomodulatory material comprising the multi-domain vesicle and an antigen.

According to still another aspect of the present invention, it is possible to provide a method for producing a multi-domain vesicle, the method including steps of: producing an oil phase solution by dissolving a first immunomodulatory material and a fluid oil in a solvent; producing a water-in-oil (W/O) emulsion by dispersing a first aqueous phase comprising a second immunomodulatory material in the oil phase solution; and mixing the water-in-oil emulsion with a second aqueous solution and evaporating the solvent, wherein the first immunomodulatory material is a fat-soluble immunostimulatory material, and the second immunomodulatory material is a water-soluble immunostimulatory material.

### [Advantageous Effects]

The present invention can provide an immunomodulatory multi-domain vesicle having a micro-sized capsule morphology, in which a plurality of liposomes including an immunomodulatory material as a basic component are connected with each other while forming respective domains, and the structural stability of the plurality of liposomes connected by the introduced fluid oil component is improved.

Further, the immunomodulatory composition according to the present invention overcomes the disadvantages of low encapsulation efficiency and short effective duration time of a single liposomal material used as various pharmaceutical compositions, and has an advantage in that an effective duration time of the immunomodulatory effect can be increased.

Moreover, the method for producing a multi-domain vesicle according to the present invention has advantages in that the stability and storage stability in the production process of the multi-domain vesicle can be improved by introducing a fluid oil such as squalene instead of triolein which was introduced in order to maintain the structural stability of a multi-liposome in the related art, the introduction of the fluid oil enables representative poorly-soluble immunomodulatory materials insoluble in a general organic solvent to be easily solubilized, and accordingly, a multi-domain vesicle comprising the various poorly-soluble immunomodulatory materials can be produced.

In addition, the multi-domain vesicle according to the present invention can increase the encapsulation efficiency and effective duration time of antigens and immunomodulatory materials with opposite charge characteristics by modulating the surface charge of the multi-domain vesicle, and various anionic or negatively charged immunomodulatory materials and biomaterials such as DNA and RNA, can be effectively loaded into the multi-domain vesicle by including a cationic lipid to constitute the multi-domain vesicle.

Moreover, since antigens and/or immunomodulatory materials loaded onto the outer wall of and inside the multi-domain vesicle are released while disintegration slowly occurs from the outer wall of the multi-domain vesicle to the inner membrane, there is an advantage in that the effective duration time of antigens and immunomodulatory materials can be increased.

Meanwhile, the multi-domain vesicle according to the present invention can increase the effective duration time of the immunomodulatory material by loading various immunomodulatory materials having lipophilic properties onto the membrane of a liposome and/or the outer wall of the multi-domain vesicle, can increase the effective duration time of the immunomodulatory material by loading various immunomodulatory materials having hydrophilic properties inside liposomes, and can increase the effective duration time of the immunomodulatory material by simultaneously loading various immunomodulatory materials having hydrophilic properties inside liposomes and a lipophilic immunomodulation material onto the membrane of liposomes and/or the outer wall of the vesicle.

Furthermore, the multi-domain vesicle according to the present invention can allow a surfactant to be coated on the outside of the multi-domain vesicle, thereby stably dispersing the multi-domain capsule in an aqueous solution.

### [Description of Drawings]

FIG. 1 is a schematic view illustrating the structure of an immune function-modulatory multi-domain vesicle (imMDV) in an embodiment of the present invention.
FIGS. 2(A) to (D) are an optical microscope image (A) and a graph (C) illustrating a size distribution of a multi-domain vesicle comprising squalene, and an optical microscope image (B) and a graph (D) illustrating a size distribution of a multi-domain vesicle comprising no squalene, in an embodiment of the present invention (scale bar: 20 µm).
FIGS. 3(A) to (C) are optical microscope images of a multi-domain vesicle comprising squalene in an embodiment of the present invention, and FIGS. 3(D) to (F) are optical microscope images of a multi-domain vesicle comprising no squalene in an embodiment of the present invention (scale bar: 4 µm).
FIGS. 4(A) to (D) are stability analysis results of a multi-domain vesicle in an embodiment of the present invention, microscope images of a multi-domain vesicle comprising squalene before centrifugation (A) and after centrifugation (C), and microscope images of a multi-domain vesicle comprising no squalene before centrifugation (B) and after centrifugation (D).
FIG. 5 is an optical microscope image of a multi-domain vesicle comprising squalene-based MPLA (imMDV(MPLA)) in an embodiment of the present invention.
FIG. 6 illustrates expression levels of cytokines secreted when BMDCs are treated with imMDV(SQ) in an embodiment of the present invention (a: TNF-alpha and b: IL-6).
FIG. 7 illustrates expression levels of cytokines secreted when BMDCs are treated with imMDV(MPLA) in an embodiment of the present invention (a: TNF-alpha, b: IL-6, and c: IL-12p70).
FIG. 8 is a graph illustrating the release behavior of ovalbumin (OVA) depending on whether squalene is comprised in a multi-domain vesicle loaded with a protein antigen (OVA) in an embodiment of the present invention.
FIG. 9 illustrates an immunomodulatory multi-domain vesicle, which is loaded with imiquimod (acid and base structures) that is an immunostimulatory material in an embodiment of the present invention (a: imMDV(R837-HCl) sample, b: imMDV(R837-base) sample, and c: imMDV[R837-HCl:R837-base (1:1) sample].
FIG. 10 illustrates the release behavior of R837 over time in a multi-domain vesicle (imMDV(R837-HCl)) for modulating immunity, which is loaded with imiquimod in an embodiment of the present invention.
FIG. 11 illustrates the expression levels of the IL-6 cytokine secreted when BMDCs are treated with a multi-domain vesicle (imMDV(R837-HCl)) loaded with imiquimod at different concentrations in an embodiment of the present invention.
FIG. 12A is a graph illustrating humoral immune effects (IgG, 1 week after injection) against an ovalbumin (OVA) cancer antigen with respect to a multi-domain vesicle loaded with imiquimod in an embodiment of the present invention (imMDV(R837-HCl) sample / 1: PBS, 2: OVA, 3: OVA+R837-HCl, and 4: OVA+imMDV sample).
FIG. 12B is a graph illustrating humoral immune effects (IgG, 1 week after injection) against an ovalbumin (OVA) cancer antigen with respect to a multi-domain vesicle loaded with imiquimod in an embodiment of the present invention (imMDV(R837-base) sample / 1: PBS, 2: OVA, 3: OVA+R837-HCl, and 4: OVA+imMDV sample).
FIG. 12C is a graph illustrating humoral immune effects (IgG, 1 week after injection) against an ovalbumin (OVA) cancer antigen with respect to a multi-domain vesicle loaded with imiquimod in an embodiment of the present invention (imMDV[R837-HCl:R837-base (1:1) / 1: PBS, 2: OVA, 3: OVA+R837-HCl, and 4: OVA+imMDV sample).
FIG. 13A is a graph illustrating humoral immune effects (IgG, 3 weeks after injection) against an ovalbumin (OVA) cancer antigen with respect to a multi-domain vesicle loaded with imiquimod in an embodiment of the present invention (imMDV(R837-HCl) sample / 1: PBS, 2: OVA, 3: OVA+R837-HCl, and 4: OVA+imMDV sample).
FIG. 13B is a graph illustrating humoral immune effects (IgG, 3 weeks after injection) against an ovalbumin (OVA) cancer antigen with respect to a multi-domain vesicle loaded with imiquimod in an embodiment of the present invention (imMDV(R837-base) sample / 1: PBS, 2: OVA, 3: OVA+R837-HCl, and 4: OVA+imMDV sample).
FIG. 13C is a graph illustrating humoral immune effects (IgG, 3 weeks after injection) against an ovalbumin (OVA) cancer antigen with respect to a multi-domain vesicle loaded with imiquimod in an embodiment of the present invention (imMDV[R837-HCl:R837-base (1:1) sample / 1: PBS, 2: OVA, 3: OVA+R837-HCl, and 4: OVA+imMDV sample).
FIG. 14A is a graph illustrating humoral immune effects (IgG, 5 weeks after injection) against an OVA cancer antigen with respect to a multi-domain vesicle loaded with imiquimod in an embodiment of the present invention (imMDV(R837-HCl) sample, 1: PBS, 2: OVA, 3: OVA+R837-HCl, and 4: OVA+imMDV).
FIG. 14B is a graph illustrating humoral immune effects (IgG, 5 weeks after injection) against an OVA cancer antigen with respect to a multi-domain vesicle loaded with imiquimod in an embodiment of the present invention (imMDV(R837-base) sample, 1: PBS, 2: OVA, 3: OVA+R837-HCl, and 4: OVA+imMDV).
FIG. 14C is a graph illustrating humoral immune effects (IgG, 5 weeks after injection) against an OVA cancer antigen with respect to a multi-domain vesicle loaded with imiquimod in an embodiment of the present invention (imMDV[R837-HCl:R837-base (1:1) sample, 1: PBS, 2: OVA, 3: OVA+R837-HCl, and 4: OVA+imMDV).
FIG. 15A is a graph illustrating humoral immune effects (IgG, 1 week after boosting of mice at week 5) against an ovalbumin (OVA) cancer antigen with respect to a multi-domain vesicle loaded with imiquimod in an embodiment of the present invention (imMDV(R837-HCl) sample / 1: PBS, 2: OVA, 3: OVA+R837-HCl, and 4: OVA+imMDV sample).
FIG. 15B is a graph illustrating humoral immune effects (IgG, 1 week after boosting of mice at week 5) against an ovalbumin (OVA) cancer antigen with respect to a multi-domain vesicle loaded with imiquimod in an embodiment of the present invention (imMDV(R837-base) sample / 1: PBS, 2: OVA, 3: OVA+R837-HCl, and 4: OVA+imMDV sample).
FIG. 15C is a graph illustrating humoral immune effects (IgG, 1 week after boosting of mice at week 5) against an ovalbumin (OVA) cancer antigen with respect to a multi-domain vesicle loaded with imiquimod in an embodiment of the present invention (imMDV[R837-HCl:R837-base (1:1) sample / 1: PBS, 2: OVA, 3: OVA+R837-HCl, and 4: OVA+imMDV sample).
FIG. 16 is a graph illustrating humoral immune effects (IgG) against an ovalbumin (OVA) cancer antigen in mice which are boosted and mice which are not boosted at week 5 after immunization of imMDV(R837-HCl)+OVA sample in an embodiment of the present invention (1: PBS, 2: OVA, 3: OVA+R837-HCl, and 4: imMDV(R837-HCl)+OVA).
FIG. 17 is a graph illustrating humoral immune effects (IgG) against an ovalbumin (OVA) cancer antigen in mice which are boosted and mice which are not boosted at week 5 after immunization of imMDV(R837-base)+OVA sample in an embodiment of the present invention (1: PBS, 2: OVA, 3: OVA+R837-HCl, and 4: imMDV(R837-base)+OVA).
FIG. 18 is a graph illustrating humoral immune effects (IgG) against an ovalbumin (OVA) cancer antigen in mice which are boosted and mice which are not boosted at week 5 after immunization of imMDV[R837-HCl:R837-base(1:1) sample]+OVA sample in an embodiment of the present invention (1: PBS, 2: OVA, 3: OVA+R837-HCl, and 4: imMDV[R837-HCl:R837-base (1:1) sample].
FIG. 19 is a graph illustrating humoral immune effects (IgG) against an ovalbumin (OVA) cancer antigen, which are sustainably shown 1, 2, and 6 weeks after the imMDV(R837-HCl)+OVA sample is immunized and boosted at week 5 in an embodiment of the present invention (1: PBS, 2: OVA, 3: OVA+R837-HCl, and 4: imMDV(R837-HCl)+OVA sample).
FIG. 20 is a graph illustrating humoral immune effects (IgG) against an ovalbumin (OVA) cancer antigen, which are sustainably shown 1, 2, and 6 weeks after the imMDV(R837-HCl)+OVA sample is immunized and boosted at week 5 in an embodiment of the present invention (1: PBS, 2: OVA, 3: OVA+R837-HCl, and 4: imMDV(R837-base)+OVA sample).
FIG. 21 is a graph illustrating humoral immune effects (IgG) against an ovalbumin (OVA) cancer antigen, which are sustainably shown 1, 2, and 6 weeks after the imMDV[R837-HCl:R837-base (1:1) sample]+OVA sample is immunized and boosted at week 5 in an embodiment of the present invention (1: PBS, 2: OVA, 3: OVA+R837-HCl, and 4: imMDV[R837-HCl:R837-base (1:1) sample).
FIG. 22 is a set of data comparing humoral immune effects (IgG) against an ovalbumin (OVA) cancer antigen shown at weeks 1 to 4 when immunizing the imMDV(R837-HCl)+OVA sample with an adjuvant in the form of an oil (DMSO(R837)+OVA) in an embodiment of the present invention (1: OVA, 2: imMDV(R837-HCl)+OVA, 3: DMSO(R837)+OVA, and 4:DMSO).
FIG. 23 is comparison of inflammatory response effects shown after immunization of two vaccines [imMDV(R837-HCl)+OVA and DMSO(R837)+OVA] in mice in an embodiment of the present invention.
FIG. 24 is a graph illustrating humoral immune effects (two weeks after intramuscular injection) of immunomodulatory materials against a hemagglutinin (HA) viral antigen in an embodiment of the present invention.
FIG. 25 is a graph illustrating humoral immune effects (four weeks after intramuscular injection) of immunomodulatory materials against a hemagglutinin (HA) viral antigen in an embodiment of the present invention.
FIG. 26 is a graph illustrating humoral immune effects of immunomodulatory materials against an ovalbumin (OVA) cancer antigen in an embodiment of the present invention.
FIG. 27 is a graph illustrating cellular immune induction effects of immunomodulatory materials against an ovalbumin (OVA) cancer antigen in an embodiment of the present invention.
FIG. 28 illustrates optical microscope images of multi-domain vesicle imMDV(SQ-Gem), imMDV(OA-Gem), and imMDV(Gem) samples in an embodiment of the present invention.
FIG. 29 is a graph confirming that loaded gemcitabine is slowly released in a multi-domain vesicle comprising squalene, whereas most of the loaded drug is released within 24 hours in a multi-domain vesicle comprising no squalene, in an embodiment of the present invention.
FIG. 30 is a graph confirming that when oleic acid vegetable oil is used instead of an animal oil such as squalene, the sustained release behavior of loaded gemcitabine exhibits a plateau shape for 24 to 72 hours, and then exhibits a linear behavior after 72 hours, in an embodiment of the present invention.
FIG. 31 is a graph illustrating imMDV(paclitaxel) and drug release behavior thereof in Example 4-2 of the present invention.
FIG. 32 illustrates imMDV(doxorubicin) in Example 4-2 of the present invention.
FIG. 33 illustrates imMDV(methotrexate) in Example 4-2 of the present invention.
FIG. 34 illustrates imMDV(oxaliplatin) in Example 4-2 of the present invention.
FIG. 35 illustrates imMDV(MK-2206) in Example 4-3 of the present invention.
FIG. 36 illustrates imMDV(PF-04691502) in Example 4-4 of the present invention.
FIG. 37 illustrates imMDV(Azacytidine) in Example 4-5 of the present invention.
FIG. 38 is a graph illustrating imMDV(Resmonostat) and drug release behavior thereof in Example 4-5 of the present invention.
FIG. 39 is a graph illustrating imMDV(Panobinostat) and drug release behavior thereof in Example 4-5 of the present invention.
FIG. 40 illustrates imMDV(OTX015(iBET)) in Example 4-5 of the present invention.
FIG. 41 illustrates imMDV(BLZ945) in Example 4-6 of the present invention.
FIG. 42 illustrates imMDV(Celecoxib) in Example 4-7 of the present invention.
FIG. 43 illustrates imMDV(GEM/R837) in Example 5 of the present invention.
FIG. 44 illustrates imMDV(BLZ945/R837) in Example 5 of the present invention.

### [Modes of the Invention]

Hereinafter, examples of the present invention will be described in detail such that those skilled in the art to which the present application pertains can easily carry out the present application with reference to the accompanying drawings. However, the present application can be implemented in various different forms, and is not limited to the embodiments described herein. In addition, in order to clearly explain the present application, portions that are not related to the explanation are omitted in the drawings, and like reference numerals are added to like portions throughout the specification.

Throughout the specification of the present application, when one part is "connected" to another part, this includes not only a case where they are "directly connected to each other", but also a case where they are "electrically connected to each other" with another element therebetween.

Throughout the specification of the present application, when one member is disposed "on" another member, this includes not only a case where the one member is brought into contact with another member, but also a case where still another member is present between the two members.

Throughout the specification of the present application, when one part "includes" one constituent element, unless otherwise specifically described, this does not mean that another constituent element is excluded, but means that the other constituent element may be further included. Throughout the specification of the present application, a term of a degree, such as "about" or "substantially", is used in a corresponding numerical value or used to mean close to the numerical value when inherent manufacturing and material allowable errors are presented in a described meaning, and is used to prevent an unconscientious infringer from illegally using disclosed contents including a numerical value illustrated as being accurate or absolute in order to help understanding of the present invention. Throughout the specification of the present application, terms, such as a "step (of performing or doing) ∼ " or a "step of ∼" does not mean a "step for ∼".

Throughout the specification of the present application, the term "combination(s) thereof' included in the Markush type expression means a mixture or combination of at least one selected from the group consisting of constituent elements described in the Markush type expression, and means including at least one selected from the group consisting of the constituent elements.

Throughout the specification of the present application, the description "A and/or B" means "A or B, or A and B".

Hereinafter, the embodiments and examples of the present invention will be described in detail with reference to the accompanying drawings. However, the present application may not be limited to the embodiments and examples and the drawings.

According to one aspect of the present invention, it is possible to provide a multi-domain vesicle comprising: at least two liposomes making contact and connected with each other, and a multi-domain vesicle outer wall surrounding the at least two liposomes. The multi-domain vesicle is formed from an oil phase and an aqueous phase, wherein the oil phase comprises a first immunomodulatory material and a fluid oil; the oil phase forms a membrane of the liposomes, and the multi-domain vesicle outer wall; the aqueous phase comprises a second immunomodulatory material; the aqueous phase is an internal aqueous phase of the membrane of the liposomes, and an outer aqueous phase of the membrane of the liposomes; the first immunomodulatory material is a fat-soluble immunostimulatory material; the second immunomodulatory material is a water-soluble immunostimulatory material; and the fluid oil improves the structural stability of the at least two liposomes making contact and connected with each other.

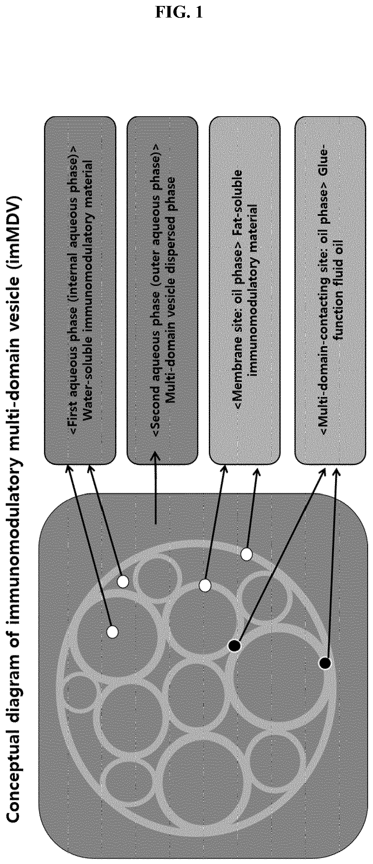

FIG. 1 is a cross-sectional view illustrating a structure of an immunomodulatory multi-domain vesicle (imMDV) according to an embodiment of the present invention. As illustrated in FIG. 1, the multi-domain vesicle may includes the out wall of the multi-domain vesicle including a fat-soluble immunostimulatory material, and may have a capsule structure with a size of about 1 µm to about 100 µm, which have at least two liposomes form each domain inside the outer wall of the multi-domain vesicle surrounding the at least two liposomes.

The multi-domain vesicle comprising the at least two liposomes may have improved duration time of an immune cell activation material, immune cell activation efficacy, encapsulation efficiency, or physiological stability as compared to a single liposome and a single emulsion in the related art.

In an embodiment of the present invention, as illustrated in FIG. 1, the inside of the membrane of the liposomes refers to an internal aqueous phase, the outside of the membrane of the liposomes refers to an external aqueous phase, and both the internal aqueous phase and the external aqueous phase mean "a first aqueous phase". The external aqueous phase, which is the outside the membrane of the liposomes, refers to a space between the membranes of the liposomes and the outer wall of the multi-domain vesicle. Further, the multi-domain vesicle may be dispersed in a solvent, and in this case, a dispersion phase in which the multi-domain vesicle is dispersed, that is, the outside of the multi-domain vesicle refers to "a second aqueous phase".

In an embodiment of the present invention, the multi-domain vesicle may have a size in a range of about 1 µm to about 100 µm, about 1 µm to about 80 µm, about 1 µm to about 60 µm, about 1 µm to about 40 µm, about 1 µm to about 20 µm, about 1 µm to about 10 µm, about 10 µm to about 100 µm, about 10 µm to about 80 µm, about 10 µm to about 60 µm, about 10 µm to about 40 µm, about 10 µm to about 20 µm, about 20 µm to about 100 µm, about 20 µm to about 80 µm, about 20 µm to about 60 µm, about 20 µm to about 40 µm, about 40 µm to about 100 µm, about 40 µm to about 80 µm, about 40 µm to about 60 µm, about 60 µm to about 100 µm, about 60 µm to about 80 µm, or about 80 µm to about 100 µm.

In an embodiment of the present invention, the multi-domain vesicle may allow the antigen and/or immunomodulatory material loaded in the vesicle to have an extended release time as compared to a single liposome or single emulsion because disintegration slowly occurs from the outer wall constituting the outer side of the vesicle to the inner membrane comprising the at least two liposomes, and as a result, it is possible to modulate the function of immune cells in vivo over a long period of time.

In an embodiment of the present invention, the at least two liposomes may include liposomes whose outer shells are in contact with each other. For example, the liposomes of the multi-domain vesicle may have improved structural stability and sustained release effects of the multi-domain vesicle because the interfacial contact between the outer shells is made and accordingly, the liposomes are not easily broken as compared to multiple liposomes where the outer shells are separated from each other.

In an embodiment of the present invention, the fluid oil may serve as a glue between domains consisting of each liposome, thereby improving the stability of the multi-domain vesicle. For example, the multi-domain vesicle may have improved stability by introducing the fluid oil onto the outer wall of the domain vesicle and making the outer walls of the liposomes come into contact with each other, and accordingly, sustained release effects and structural stability may be enhanced.

In an embodiment of the present invention, the fat-soluble immunostimulatory material may be easily loaded into the multi-domain vesicle by the fluid oil. For example, imiquimod (R837) and the like, which are poorly-soluble materials difficult to be solubilized in a general organic solvent, are easily solubilized by the fluid oil, so that the poorly-soluble material may be loaded into a space between the liposomes along with the fluid oil in the multi-domain vesicle.

In an embodiment of the present invention, the fluid oil may serve as an adjuvant that helps the activation of immune cells, and may be selected from the group consisting of, for example, an animal oil, a vegetable oil, a tocopherol, mineral oil, castor oil, and combinations thereof.

In an embodiment of the present invention, the animal oil may include a fish oil.

In an embodiment of the present invention, the fish oil may be used without limitation as long as it is a metabolizable oil, and may include, for example, cod liver oil, shark liver oil, whale oil, or the like. The shark liver oil contains squalene, a molecule known as 2,6,10,15,19,23-hexamethyl-2,6,10,14,18,22-tetracosahexaene, and an unsaturated terpene, and may also include the saturated analog squalane. A fish oil including squalene or squalane is easily available from commercial supply sources, or may be obtained by methods known in the art.

In an embodiment of the present invention, the animal-derived oil may include lard, a resin (tallow) oil, beef tallow, or the like.

In an embodiment of the present invention, the vegetable-derived oil may be an oil derived from nuts, seeds, grains, or the like, and may include, for example, peanut oil, soybean oil, coconut oil, olive oil, or the like.

In an embodiment of the present invention, the tocopherol may be a tocopherol containing vitamin E. Although there are various tocopherols (α, β, γ, δ, ε, or ξ), α-tocopherol may be generally used, and for example, DL-α-tocopherol may be used.

In an embodiment of the present invention, by introducing the fluid oil into the multi-domain vesicle, the immunomodulatory material may be easily solubilized, and the structural stability of the multi-domain vesicle may be strengthened. For example, when squalene or oleic acid is used as the fluid oil, a lipophilic or poorly-soluble immunomodulatory material may be easily solubilized, and it is possible to exhibit a synergistic effect with the immunomodulatory material by the immune activation(immune stimulation) effect of squalene and oleic acid themselves, and to increase the structural stability of the multi-domain vesicle, but the fluid oil is not limited thereto.

In an embodiment of the present invention, the fat-soluble and water-soluble immunostimulatory materials may be an immunomodulatory material expressed in cancer cells under stress, for example, a heat-shock protein, or may be a material inducing the activation of T cells.

In an embodiment of the present invention, the fat-soluble and water-soluble immunostimulatory materials may include at least one material selected from the group consisting of a toll-like receptor agonist, a saponin, an anti-viral peptide, an inflammasome inducer, an NOD ligand, a cytosolic DNA sensor (CDS) ligand, a stimulator of interferon genes (STING) ligand, and combinations thereof, but is not limited thereto.

In an embodiment of the present invention, the toll-like receptor agonist may refer to a component capable of causing a signaling response via a TLT signaling pathway by generating an endogenous or exogenous ligand as a direct ligand or as an indirect ligand.

In an embodiment of the present invention, the toll-like receptor agonist may be a natural toll-like receptor agonist or a synthetic toll-like receptor agonist.

In an embodiment of the present invention, the toll-like receptor agonist may be one capable of causing a signaling response via TLR-1, and may include at least one material selected from the group consisting of, for example, a tri-acylated lipopeptide (LP); a phenol-soluble modulin; a Mycobacterium tuberculosis lipopeptide; a bacterial lipopeptide from S-(2,3-bis(palmitoyloxy)-(2-RS)-propyl)-N-palmitoyl-(R)-Cys-(S)-Ser-(S)-Lys(4)-OH; a bacterial lipopeptide from Borrelia burgdorfei; a trihydrochloride (Pam3Cys) lipopeptide that mimics an acetylated amino terminal of an OspA lipopeptide; and combinations thereof, but is not limited thereto.

In an embodiment of the present invention, the toll-like receptor agonist may include a TLR-2 agonist, and may include, for example, Pam3Cys-Lip, but is not limited thereto.

In an embodiment of the present invention, the toll-like agonist may include a TLR-3 agonist, and may include, for example, Poly(I:C), Poly(ICLC), Poly(IC12U), Ampligen, and the like as a Poly(I:C)-series, but is not limited thereto.

In an embodiment of the present invention, the toll-like agonist may include a TLR-4 agonist, and may include at least one material selected from the group consisting of, for example, a Shigella flexneri outer membrane protein preparation, AGP, CRX-527, MPLA, PHAD, 3D-PHAD, GLA, and combinations thereof, but is not limited thereto.

In an embodiment of the present invention, the toll-like receptor agonist may include a TLR-5 agonist, and may include, for example, flagellin or a fragment thereof, but is not limited thereto.

In an embodiment of the present invention, the toll-like receptor agonist may include a TLR-7 agonist or a TLR-8 agonist, and may include at least one material selected from the group consisting of, for example, imiquimod, R837, resquimod, or an imidazoquinoline molecule such as R848; VTX-2337; CRX642; imidazoquinoline covalently bonded to a phospholipid group or a phosphonolipid group; and combinations thereof, but is not limited thereto.

In an embodiment of the present invention, the toll-like receptor agonist may include a TLR-9 agonist, and may include, for example, an immunostimulatory oligonucleotide, but is not limited thereto.

In an embodiment of the present invention, the immunostimulatory oligonucleotide may include at least one CpG motif, but is not limited thereto.

In an embodiment of the present invention, the saponin may be selected from the group consisting of QS21, Quil A, QS7, QS17, β-escin, digitonin, and combinations thereof, but is not limited thereto.

In an embodiment of the present invention, the anti-viral peptide may include KLK, but is not limited thereto.

In an embodiment of the present invention, the inflammasome inducer may be trehalose-6,6-dibehenate (TDB), but is not limited thereto.

In an embodiment of the present invention, the NOD ligand may be an NOD2 agonist-synthetic muramyl tripeptide (M-TriLYS) or N-glycosylated muramyl dipeptide (NOD2 agonist), but is not limited thereto.

In an embodiment of the present invention, the CDS ligand may be Poly(dA:dT), but is not limited thereto.

In an embodiment of the present invention, the STING ligand may be cGAMP, di-AMP, or di-GMP, but is not limited thereto.

In an embodiment of the present invention, the immunomodulatory material may include a combination of one or two or more toll-like receptor agonists, and may include a dual TLR2 and TLR7 agonist (CL401) or a dual TLR2 and NOD2 agonist (CL429), but is not limited thereto.

In an embodiment of the present invention, the immunomodulatory material included in the multi-domain vesicle may be selected from the group consisting of, for example, Pam3Cys-Lip, Poly(I:C), CRX-527, MPLA, flagellin, imiquimod, resquimod, CpG, QS21, M-MurNAc-Ala-D-isoGln-Lys (M-TriLys), trehalose-6,6-dibehenate (TDB), 8837, Poly(dA:dT), cGAMP, and combinations thereof, but is not limited thereto.

In an embodiment of the present invention, the fat-soluble immunostimulatory material may include a material selected from the group consisting of, for example, a cationic lipid, MPLA, AGP, CRX-527, PHAD, 3D-PHAD, GLA, a lipid peptide, Pam3Cys, Pam3Cys-Lip, DDA, imiquimod (base form), resquimod (base form), VTX-2337, CRX642, a saponin (QS21), TDB, CL401, CL429, and combinations thereof.

In an embodiment of the present invention, the hydrophilic immunostimulatory material may include a material selected from the group consisting of, for example, CpG, imiquimod (HCl form), resquimod (HCl form), Poly(I:C), STING, flagellin, a saponin, a KLK peptide, an NOD agonist peptide, Poly(dA:dT), and combinations thereof. For example, the hydrophilic material may be conjugated to the outer wall of the multi-domain vesicle even through a chemical bonding group of a terminal group, but is not limited thereto.

In an embodiment of the present invention, an electrostatic attraction force with a cellular membrane that is anionic is induced by the cationic lipid, so that the intracellular delivery efficiency of the immunomodulatory material may be further improved.

According to an embodiment of the present invention, various anionic or negatively charged immunomodulatory materials and biomaterials such as DNA and RNA, may be effectively loaded into the multi-domain vesicle by including a cationic lipid to constitute the multi-domain vesicle. For example, anionic or negatively charged biomaterials and/or immunomodulatory materials based on DNA or RNA amino acids may be loaded onto the outer wall of the multi-domain vesicle or the membrane of internal liposomes, which exhibits cationic characteristics, through an electrostatic bond, but is not limited thereto.

In an embodiment of the present invention, the cationic lipid may include a material selected from the group consisting of 3β-[N-(N',N'-dimethylaminoethane)-carbamoyl]cholesterol hydrochloride (DC-cholesterol), dimethyldioctadecylammonium (DDA), 1,2-dioleoyl-3-trimethylammonium-propane (DOTAP), 1,2-di-O-octadecenyl-3-trimethylammonium propane (DOTMA), 1,2-dimyristoleoyl-sn-glycero-3-ethylphosphocholine (EPC), N1-[2-((1S)-1-[(3-aminopropyl)amino] -4- [di(3 -amino-propyl)amino]butylcarboxamido)ethyl] -3,4-di[oleyloxy]-benzamide (MVL5), the lipid 1,2-dioleoyl-3-dimethylammonium-propane (DODAP), and combinations thereof, but is not limited thereto.

According to an embodiment of the present invention, a surfactant is coated onto the outside of a multi-domain vesicle, so that the multi-domain vesicle may be stably dispersed in an aqueous solution.

The surfactant is coated onto the outside of the multi-domain vesicle, thereby allowing the multi-domain vesicle to be dispersed in an aqueous solution, and for example, a polyoxyethylene sorbitan ester surfactant (generally called Tween), in particular, Polysorbate 20 and Polysorbate 80; a copolymer of ethylene oxide (EO), propylene oxide (PO), and/or butylene oxide (BO); octoxynol (for example, Triton X-100, or t-octylphenoxypolyethoxyethanol); (octylphenoxy)polyethoxyethanol (IGEPAL CA-630/NP-40); as a phospholipid (a phospholipid component), phosphatidylcholine (lecithin) phosphatidylethanol aniline, phosphatidylserine, phosphatidylinositol, phosphatidylglycerol, phosphatidic acid, sphingomyelin, and cardiolipin; a nonylphenol ethoxylate such as the Tergitol™ NP series; a polyoxyethylene fatty ether derived from lauryl, cetyl, and oleyl alcohols (known as a Brij surfactant) such as triethylene glycol monolauryl ether (Brij 30); and a sorbitan ester (generally known as SPAN) such as sorbitan trioleate (Span85) and sorbitan monolaurate may be used either alone or in a combination of at least two surfactants. For example, as the surfactant, a mixture of these surfactants, for example, a Tween 80/Span 85 mixture may be used. A combination of polyoxyethylene sorbitan ester and octoxynol may also be used. Another useful combination may include laureth 9, a polyoxyethylene sorbitan ester and/or octoxynol. The surfactant may be used at a content of 0.001 to 20 wt% based on the total weight of the entire multi-domain vesicle, and may be used at a weight of, for example, 0.01 to 1 wt%, 0.001 to 0.1 wt%, 0.005 to 0.02 wt%; 0.1 to 20 wt%, 0.1 to 10 wt%, 0.1 to 1 wt%, or about 0.5 wt%.

According to another aspect of the present invention, provided is an immunomodulatory material including a multi-domain vesicle according to the present invention and an antigen.

In an embodiment of the present invention, the antigen may be selected from the group consisting of a protein, a gene, a cell, a virus, and combinations thereof, but is not limited thereto. For example, the protein may include ovalbumin, a recombinant protein, a subunit, and a split protein antigen, the cell may include, for example, a dendritic cell and a T cell, and the virus may include, for example, influenza, hepatitis B virus (HBV), hepatitis A virus (HAV) and human papillomavirus (HPV), but is not limited thereto.

In an embodiment of the present invention, the antigen may be selected from the group consisting of an attenuated live complete body microorganism, an inert microorganism, a ruptured microorganism, a protein of a pathogen, a recombinant protein, a sugar protein, a peptide, polysaccharides, lipopolysaccharides, a lipopeptide, a polynucleotide, a cell, a virus, and combinations thereof, but is not limited thereto. For example, the antigen may include an influenza-derived antigen or a cancer cell-derived antigen, but is not limited thereto. For example, the immunomodulatory material for intradermal administration may include at least one antigen to induce multiple in vivo immune responses, but is not limited thereto.

In an embodiment of the present invention, the cancer cell may be obtained using a cancer cell line, or may be isolated from a cancer tissue (tumor tissue) present in the body. Further, the cancer call may be produced by applying an anticancer drug or radiation to an actual cancer tissue to induce the production of a protein related to intracellular stress, and then dissolving cancer cells, but the method is not limited thereto.

In an embodiment of the present invention, the cancer cell may include cancer cells of the lungs, colon, central nervous system, skin, ovaries, kidneys, breasts, stomach, or large intestine, but is not limited thereto.

According to still another aspect of the present invention, provided is a method for producing a multi-domain vesicle, the method including steps of: producing an oil phase solution by dissolving a first immunomodulatory material and a fluid oil in a solvent; producing a water-in-oil (W/O) emulsion by dispersing a first aqueous phase comprising a second immunomodulatory material in the oil phase solution; and mixing the water-in-oil emulsion with a second aqueous solution and evaporating the solvent, wherein the first immunomodulatory material is a fat-soluble immunostimulatory material, and the second immunomodulatory material is a water-soluble immunostimulatory material.

In an embodiment of the present invention, the multi-domain vesicle may allow the antigen and/or immunomodulatory material loaded in the vesicle to have an extended release time as compared to a single liposome or single emulsion because disintegration slowly occurs from the outer wall constituting the outer side of the vesicle to the inner membrane comprising the at least two liposomes, and as a result, it is possible to modulate the function of immune cells in vivo over a long period of time.

In an embodiment of the present invention, the at least two liposomes may include liposomes whose outer shells are in contact with each other. For example, the liposomes of the multi-domain vesicle may have improved structural stability and sustained release effects of the multi-domain vesicle because the interfacial contact between the outer shells is made and accordingly, the liposomes are not easily broken as compared to multiple liposomes which the outer shells are separated from each other.

In an embodiment of the present invention, the fluid oil serves as a glue between domains consisting of each liposome, and thus is characterized by improving the stability of the multi-domain vesicle. For example, the multi-domain vesicle may have improved stability by introducing the fluid oil onto the outer wall of the domain vesicle and making the outer walls of the liposomes come into contact with each other, and accordingly, sustained release effects and structural stability may be enhanced.

In an embodiment of the present invention, the lipophilic immunostimulatory material may be easily loaded into the multi-domain vesicle by the fluid oil. For example, imiquimod (R837) and the like, which are poorly-soluble materials difficult to be solubilized in a general organic solvent, are easily solubilized by the fluid oil, so that the poorly-soluble material may be loaded into a space between the liposomes along with the fluid oil in the multi-domain vesicle.

In an embodiment of the present invention, the fluid oil may serve as an adjuvant that helps the activation of immune cells, and may be selected from the group consisting of, for example, an animal oil, a vegetable oil, tocopherol, a mineral oil, castor oil, and combinations thereof.

In an embodiment of the present invention, the animal oil may include a fish oil.

In an embodiment of the present invention, the fish oil may be used without limitation as long as it is a metabolizable oil, and may include, for example, cod liver oil, shark liver oil, whale oil, or the like. The shark liver oil contains squalene, a molecule known as 2,6,10,15,19,23-hexamethyl-2,6,10,14,18,22-tetracosahexaene, and an unsaturated terpene, and may also include the saturated analog squalane. A fish oil including squalene or squalane is easily available from commercial supply sources, or may be obtained by methods known in the art.

In an embodiment of the present invention, the animal-derived oil may include lard, a resin (tallow) oil, beef tallow, or the like.

In an embodiment of the present invention, the vegetable-derived oil may be an oil derived from nuts, seeds, grains, or the like, and may include, for example, peanut oil, soybean oil, coconut oil, olive oil, or the like.

In an embodiment of the present invention, the tocopherol may be a tocopherol containing vitamin E. Although there are various tocopherols (α, β, γ, δ, ε, or ξ), α-tocopherol may be generally used, and for example, DL-α-tocopherol may be used.

In an embodiment of the present invention, by introducing the fluid oil into the multi-domain vesicle, the immunomodulatory material may be easily solubilized, and the structural stability of the multi-domain vesicle may be strengthened. For example, when squalene or oleic acid is used as the fluid oil, a lipophilic or poorly-soluble immunomodulatory material may be easily solubilized, and it is possible to exhibit a synergistic effect with the immunomodulatory material by the immune activation effect of squalene and oleic acid themselves, and to increase the structural stability of the multi-domain vesicle, but the fluid oil is not limited thereto.

In an exemplary embodiment of the present invention, the fat-soluble and water-soluble immunostimulatory materials may be an immunomodulatory material expressed in cancer cells under stress, for example, a heat-shock protein, or may be a material inducing the activation of T cells.

In an embodiment of the present invention, the fat-soluble and water-soluble immunostimulatory materials may include at least one material selected from the group consisting of a toll-like receptor agonist, a saponin, an anti-viral peptide, an inflammasome inducer, an NOD ligand, a cytosolic DNA sensor (CDS) ligand, a stimulator of interferon genes (STING) ligand, and combinations thereof, but is not limited thereto.

In an embodiment of the present invention, the toll-like receptor agonist may refer to a component capable of causing a signaling response via a TLT signaling pathway by generating an endogenous or exogenous ligand as a direct ligand or as an indirect ligand.

In an embodiment of the present invention, the toll-like receptor agonist may be a natural toll-like receptor agonist or a synthetic toll-like receptor agonist.

In an embodiment of the present invention, the toll-like receptor agonist may be one capable of causing a signaling response via TLR-1, and may include at least one material selected from the group consisting of, for example, a tri-acylated lipopeptide (LP); a phenol-soluble modulin; a Mycobacterium tuberculosis lipopeptide; a bacterial lipopeptide from S-(2,3-bis(palmitoyloxy)-(2-RS)-propyl)-N-palmitoyl-(R)-Cys-(S)-Ser-(S)-Lys(4)-OH; a bacterial lipopeptide from Borrelia burgdorfei; a trihydrochloride (Pam3Cys) lipopeptide that mimics an acetylated amino terminal of an OspA lipopeptide; and combinations thereof, but is not limited thereto.

In an embodiment of the present invention, the toll-like receptor agonist may include a TLR-2 agonist, and may include, for example, Pam3Cys-Lip, but is not limited thereto.

In an embodiment of the present invention, the toll-like agonist may include a TLR-3 agonist, and may include, for example, Poly(I:C), Poly(ICLC), Poly(IC12U), Ampligen, and the like as a Poly(I:C)-series, but is not limited thereto.

In an embodiment of the present invention, the toll-like agonist may include a TLR-4 agonist, and may include at least one material selected from the group consisting of, for example, a Shigella flexneri outer membrane protein preparation, AGP, CRX-527, MPLA, PHAD, 3D-PHAD, GLA, and combinations thereof, but is not limited thereto.

In an embodiment of the present invention, the toll-like receptor agonist may include a TLR-5 agonist, and may include, for example, flagellin or a fragment thereof, but is not limited thereto.

In an embodiment of the present invention, the toll-like receptor agonist may include a TLR-7 agonist or a TLR-8 agonist, and may include at least one material selected from the group consisting of, for example, imiquimod, R837, resquimod, or an imidazoquinoline molecule such as R848; VTX-2337; CRX642; imidazoquinoline covalently bonded to a phospholipid group or a phosphonolipid group; and combinations thereof, but is not limited thereto.

In an embodiment of the present invention, the toll-like receptor agonist may include a TLR-9 agonist, and may include, for example, an immunostimulatory oligonucleotide, but is not limited thereto.

In an embodiment of the present invention, the immunostimulatory oligonucleotide may include at least one CpG motif, but is not limited thereto.

In an embodiment of the present invention, the saponin may be selected from the group consisting of QS21, Quil A, QS7, QS17, β-escin, digitonin, and combinations thereof, but is not limited thereto.

In an embodiment of the present invention, the anti-viral peptide may include KLK, but is not limited thereto.

In an embodiment of the present invention, the inflammasome inducer may be trehalose-6,6-dibehenate (TDB), but is not limited thereto.

In an embodiment of the present invention, the NOD ligand may be an NOD2 agonist-synthetic muramyl tripeptide (M-TriLYS) or N-glycosylated muramyl dipeptide (NOD2 agonist), but is not limited thereto.

In an embodiment of the present invention, the CDS ligand may be Poly(dA:dT), but is not limited thereto.

In an embodiment of the present invention, the STING ligand may be cGAMP, di-AMP, or di-GMP, but is not limited thereto.

In an embodiment of the present invention, the immunomodulatory material may include a combination of one or two or more toll-like receptor agonists, and may include a dual TLR2 and TLR7 agonist (CL401) or a dual TLR2 and NOD2 agonist (CL429), but is not limited thereto.

In an embodiment of the present invention, the immunomodulatory material included in the multi-domain vesicle may be selected from the group consisting of, for example, Pam3Cys-Lip, Poly(I:C), CRX-527, MPLA, flagellin, imiquimod, resquimod, CpG, QS21, M-MurNAc-Ala-D-isoGln-Lys (M-TriLys), trehalose-6,6-dibehenate (TDB), 8837, Poly(dA:dT), cGAMP, and combinations thereof, but is not limited thereto.

In an embodiment of the present invention, the fat-soluble immunostimulatory material may include a material selected from the group consisting of, for example, a cationic lipid, MPLA, AGP, CRX-527, PHAD, 3D-PHAD, GLA, a lipid peptide, Pam3Cys, Pam3Cys-Lip, DDA, imiquimod (base form), resquimod (base form), VTX-2337, CRX642, a saponin (QS21), TDB, CL401, CL429, and combinations thereof.

In an embodiment of the present invention, the hydrophilic immunomodulatory material may include a material selected from the group consisting of, for example, CpG, imiquimod (HCl form), resquimod (HCl form), Poly(I:C), STING, flagellin, a saponin, a KLK peptide, an NOD agonist peptide, Poly(dA:dT), and combinations thereof. For example, the hydrophilic material may be conjugated to the outer wall of the multi-domain vesicle even through a chemical bonding group of a terminal group, but is not limited thereto.

In an embodiment of the present invention, by the cationic lipid, an electrostatic attraction force with a cellular membrane which is anionic is induced, so that the intracellular delivery efficiency of the immunomodulatory material may be further improved.

According to an embodiment of the present invention, by including a cationic lipid, various anionic and/or negatively charged immunomodulatory materials and biomaterials such as DNA and RNA, may be effectively loaded into the multi-domain vesicle to constitute the multi-domain vesicle. For example, anionic or negatively charged biomaterials and/or immunomodulatory materials based on DNA or RNA amino acids may be loaded onto the outer wall of the multi-domain vesicle or the membrane of internal liposomes, which exhibits cationic characteristics through an electrostatic bond, but is not limited thereto.

In an embodiment of the present invention, the cationic lipid may include a material selected from the group consisting of 3β-[N-(N',N'-dimethylaminoethane)-carbamoyl]cholesterol hydrochloride (DC-cholesterol), dimethyldioctadecylammonium (DDA), 1,2-dioleoyl-3-trimethylammonium-propane (DOTAP), 1,2-di-O-octadecenyl-3-trimethylammonium propane (DOTMA), 1,2-dimyristoleoyl-sn-glycero-3-ethylphosphocholine (EPC), N1-[2-((1S)-1-[(3-aminopropyl)amino] -4- [di(3 -amino-propyl)amino]butylcarboxamido)ethyl] -3,4-di[oleyloxy]-benzamide (MVL5), the lipid 1,2-dioleoyl-3-dimethylammonium-propane (DODAP), and combinations thereof, but is not limited thereto.

According to an embodiment of the present invention, a surfactant is coated onto the outside of a multi-domain vesicle, so that the multi-domain vesicle may be stably dispersed in an aqueous solution.

The surfactant is coated onto the outside of the multi-domain vesicle, thereby allowing the multi-domain vesicle to be dispersed in an aqueous solution, and for example, a polyoxyethylene sorbitan ester surfactant (generally called Tween), in particular, Polysorbate 20 and Polysorbate 80; a copolymer of ethylene oxide (EO), propylene oxide (PO), and/or butylene oxide (BO); octoxynol (for example, Triton X-100, or t-octylphenoxypolyethoxyethanol); (octylphenoxy)polyethoxyethanol (IGEPAL CA-630/NP-40); as a phospholipid (a phospholipid component), phosphatidylcholine (lecithin) phosphatidylethanol aniline, phosphatidylserine, phosphatidylinositol, phosphatidylglycerol, phosphatidic acid, sphingomyelin, and cardiolipin; a nonylphenol ethoxylate such as the Tergitol™ NP series; a polyoxyethylene fatty ether derived from lauryl, cetyl, and oleyl alcohols (known as a Brij surfactant) such as triethylene glycol monolauryl ether (Brij 30); and a sorbitan ester (generally known as SPAN) such as sorbitan trioleate (Span85) and sorbitan monolaurate may be used either alone or in a combination of at least two surfactants.

For example, as the surfactant, a mixture of these surfactants, for example, a Tween 80/Span 85 mixture may be used. A combination of polyoxyethylene sorbitan ester and octoxynol may also be used. Another useful combination may include laureth 9, a polyoxyethylene sorbitan ester and/or octoxynol. The surfactant may be used at a content of 0.001 to 20 wt% based on the total weight of the entire multi-domain vesicle, and may be used at a weight of, for example, 0.01 to 1 wt%, 0.001 to 0.1 wt%, 0.005 to 0.02 wt%; 0.1 to 20 wt%, 0.1 to 10 wt%, 0.1 to 1 wt%, or about 0.5 wt%.

According to another aspect of the present invention, it is possible to provide the multi-domain vesicle comprising: at least two liposomes making contact and connected with each other, and a multi-domain vesicle outer wall surrounding the at least two liposomes. The multi-domain vesicle is formed from an oil phase and an aqueous phase, wherein the oil phase comprises a first immunomodulatory material and a fluid oil; the oil phase forms a membrane of the liposomes, and the multi-domain vesicle outer wall; the aqueous phase comprises a second immunomodulatory material; the aqueous phase is an internal aqueous phase of the membrane of the liposomes, and an outer aqueous phase of the membrane of the liposomes; the first immunomodulatory material and the second immunomodulatory material are immunosuppressive factor control materials; and the fluid oil improves the structural stability of the at least two liposomes making contact and connected with each other.

The first immunomodulatory material and the second immunomodulatory material may further include the above-described immunostimulatory material. That is, the first immunomodulatory material and the second immunomodulatory material may include an immunosuppressive factor control material along with the immunostimulatory material.

Moreover, according to still another aspect of the present invention, it is possible to provide an immunomodulatory material comprising the multi-domain vesicle and an antigen.

Further, it is possible to provide a method for producing a multi-domain vesicle, the method including steps of: producing an oil phase solution by dissolving a first immunomodulatory material and a fluid oil in a solvent; producing a water-in-oil (W/O) emulsion by dispersing a first aqueous phase comprising a second immunomodulatory material in the oil phase solution; and mixing the water-in-oil emulsion with a second aqueous solution and evaporating the solvent, wherein the first immunomodulatory material and the second immunomodulatory material are immunosuppressive factor control materials.

In the present invention, a multi-domain vesicle-based solid cancer microenvironment control composition is a new form of an immunomodulatory composition for modulating the microenvironment of cancer, and is characterized by including a drug (immunosuppressive factor control material) capable of controlling the functions of an immunosuppressive cell and an immunosuppressive material appearing in the solid cancer microenvironment in addition to the previously mentioned material that activates the in vivo immune cells.

According to an embodiment of the present invention, it is possible to produce an immunomodulatory multi-domain vesicle having a micro-sized capsule morphology, in which a plurality of liposomes including an immunosuppressive factor control material capable of controlling the functions of an immunosuppressive factor, that is, an immunosuppressive cell and an immunosuppressive material as a basic component, are connected with each other while forming respective domains, and the structural stability of the plurality of liposomes connected by the introduced fluid oil component is improved. Further, according to an embodiment of the present invention, it is possible to produce an anti-cancer therapeutic agent composition based on a new multi-domain vesicle, which may overcome the disadvantages of low encapsulation efficiency and short effective duration time of a single liposomal material used as various pharmaceutical compositions, and increase an effective duration time of the immune function modulatory effect.

The multi-domain vesicle according to an embodiment of the present invention has an advantage in that an effective duration time of an immune machinery modulatory material may be increased because an immunosuppressive factor control material capable of controlling the functions of an immunosuppressive cell and an immunosuppressive material loaded onto the outer wall of and inside the vesicle is released while disintegration slowly occurs from the outer wall of the vesicle to the inner membrane.

In addition, the multi-domain vesicle according to an embodiment of the present invention may increase the effective duration time of an immunostimulatory material by loading an immunosuppressive factor control material capable of controlling the functions of various immunosuppressive cells and immunosuppressive materials having lipophilic properties onto the membrane of liposomes and/or the outer wall of the multi-domain vesicle.

The multi-domain vesicle according to an embodiment of the present invention may increase the effective duration time of an immunosuppressive factor control material by loading the immunosuppressive factor control material capable of controlling the functions of various immunosuppressive cells and immunosuppressive materials having hydrophilic properties inside the liposomes.

The multi-domain vesicle according to an embodiment of the present invention may increase the effective duration time of an immunosuppressive factor control material capable of controlling the functions of an immunosuppressive cell and an immunosuppressive material by simultaneously loading various immunosuppressive factor control materials having hydrophilic properties inside the liposomes and a lipophilic immunosuppressive factor control material onto the membrane of liposomes and/or the outer wall of the vesicle.

In an example of the present invention, examples of a drug capable of controlling the function of myeloid-derived suppressor cells (MDSCs), that is, an immunosuppressive factor control material, include Tadalafil, Sildenafil, L-AME, Nitroaspirin, Celecoxib, NOHA, Bardoxolone methyl, D,L-1-methyl-tryptophan, 5-Fluorouracil, Gemcitabine, 17-DMAG, Peptide-Fc fusion proteins, ATRA, Vitamin A, Vitamin D3, Vitamin E, GR1 antibodies, Zoledronic acid, Sunitinib, Axitinib, Decetaxel, Sorafenib, Cucurbitacin B, JSI-124, Anti-IL-17 antibodies, Anti-glycan antibodies, Anti-VEGF antibodies, Bevacizumab, Antracycline, Tasquinimod, Imatinib, and cyclophosphamide, but are not limited thereto.

In an example of the present invention, a PI3K inhibitor includes PX-866, Wortmannin, PI-103, Pictilisib, GDC-0980, PF-04691502, BEZ235, XL765, XL147, BAY80-6946, GSK-2126458, Buparlisib, BYL719, AZD8186, GSK-2636771, CH5132799, INK-1117, and the like.

In an example of the present invention, a PI3K delta inhibitor material includes AMG-319, Idelalisib, TRG-1202, INCB050465, IPI-145, Duvelisib, Acalisib, TG-1202, RV1729, RP-6530, GDC-0032, and the like.

In an example of the present invention, a PI3K gamma inhibitor material includes IPI-549, IPI-145, and the like.

In an example of the present invention, examples of a drug capable of controlling the function of regulatory T cells (Treg), that is, an immunosuppressive factor control material, include Anti-CD25 antibodies (daclizumab), Basiliximab, LMB-2, Denileukin diftitox(Ontak), Bivalent IL-2 fusion toxin, Anti-TGF-beta antibodies, fresolimumab, TGF-betaR kinase inhibitors, LY2157299, Soluble TGF-betaR I/II, Ipilimumab, Tremelimumab, Pembrolizumab, Nivolumab, TIM-3 antibodies, LAG-3 antibodies, Anti-CD39 antibodies, Anti-73 antibodies, A(2A)R inhibitors, Celecoxib, Indomethacin, Diclofenac, Ibuprofen, TNFR2 antibodies, Anti-GITR antibodies, Bevacizumab, Anti-OX40(CD134) antibodies, soluble GITR ligand, Blockades for chemokine receptors (CCR4, 5, 6,10), cyclophosphamide, Sunitinib, Fludarabine, PI3K p110(delta) inhibitors, CliniMACs, Mogamulizumab, Fingolimod, Regulators for miRNA (miR-155, miR-146a, miR-181a), 5-aza-2-deoxycytidine, paclitaxel, Imatinib, Sorafenib, Cyclosporin A, Tacrolimus, Dasatinib, Poly-G-oligonucleotide, TLR8 ligands, gemcitabine, and 5-fluorouracil, but are not limited thereto.

In an example of the present invention, a drug capable of modulating the function of tumor associated macrophages (TAMs), that is, an immunosuppressive factor control material, is a drug capable of inhibiting the recruitment of a macrophage, and includes CCL2/CCR2 inhibitors (Yondeli, RS102895), M-CSF or M-CSFR inhibitors (anti-M-CSF antibodies, JNJ-28312141, GW2580), chemoattractants (CCL5, CXCL-12, VEGF) and inhibitors for receptors thereof, HIFs inhibitors, and the like, but is not limited thereto.

In addition, a drug capable of inhibiting the survival of TAMs, that is, an immunosuppressive factor control material, includes a drug capable of inducing the expression of bisphosphonates, Clodronate, Dasatinib, anti-FR beta antibodies, Shigella flexneri, Legumain, and CD1d, but is not limited thereto.

Moreover, a drug capable of improving the characteristics of the M1 macrophage, that is, an immunosuppressive factor control material, includes a TLR agonist that is an NF-kB agonist, Anti-CD40 antibodies, Thiazolidinediones, Tasquinimod, Anti-IL-10R antibodies, Anti-IL-10 antibodies, an oligonucleotide (Anti-IL-10R Anti-IL-10), an interferon that is an STAT1 agonist, SHIP capable of inducing the M1 pathway, GM-CSF, IL-12, Thymosin alpha1, and the like, but is not limited thereto.

Furthermore, a drug capable of inhibiting the machinery for helping the growth of cancer cells based on the M2 macrophage, that is, an immunosuppressive factor control material, includes sunitinib, sorafenib, WP1066, corosolic acid, oleanolic acid which are STAT3 inhibitors, STAT6 inhibitors, M2 pathway (c-Myc, PPAR-alpha/gamma, PI3K, KLF4, HIFs, Ets2, DcR3, and mTOR) inhibitors, HRG, CuNG, MDXAA, Silibinin, and PPZ, but is not limited thereto.

Moreover, a target miRNA capable of controlling the function of a macrophage in a tumor microenvironment includes miR-155, miR-511-3p, and miR-26a.

Moreover, a target drug capable of enhancing the anticancer efficacy by targeting the macrophage in the tumor microenvironment includes Paclitaxel, Docetaxel, 5-Flurouracil, Alendronate, Doxorubicin, Simvastatin, Hydrazinocurcumin, Amphotericin B, Ciprofloxacin, Rifabutin, Rifampicin, Efavirenz, Cisplatin, Theophyline, Pseudomonas exotoxin A, Zoledronic acid, Trabectedin, Siltuximab (Anti-IL-6 antibodies), Dasatinib, CpG-ODN, Interferon-alpha, -beta, -gamma, GM-CSF, IL-12, Thymosin alpha-1, Sunitinib, 5,6-Dimethylxanthenone-4-acetic acid, Silibinin, CCL2-CCR2 inhibitors (PF-04136309, Trabectedin, Carlumab), a ligand (imiquimod, 852A) of a CSF1-CSF1R signaling blocker (BLZ945, PLX3397, Emactuzumab (RG7155), AMG-820, IMC-CS4, GW3580, and PLX6134) and a toll-like receptor 7, NF-kB inhibitors (N-acetyl-1-cystein, Vitamin C, bortezomib, aspirin, salicylates, Indolecarboxamide derivatives, quinazoline analogs, Thalidomide, prostaglandin metabolites), HIF-1 inhibitors (2ME2, 17-AAG, Camptothecin, Topotecan, Pleurotin, 1-methylpropyl, 2-imidazolyl disulfide, and YC-1), a CXCR4 agonist (AMD3100, AMD1498), ALX40-4C, T22, T140, CGP64222, and KRH-1636, but is not limited thereto.

An example of the present invention may provide a composition based on a multi-domain vesicle containing an immunosuppressive environmental factor suppressor (Transforming growth factor beta (TGF-beta) inhibitors, Nitro aspirin, Cycloxygenase-2(COX2) inhibitors, Indoleamine 2,3-dioxygenase (IDO) inhibitors, Phosphodiesterase-5 (PDE-5) inhibitors, and Anti-Interleukin 10 (IL-10)) drugs.

In an example of the present invention, a TGF-beta inhibitor includes SB-505124, LY-364974, and the like, but is not limited thereto.

In an example of the present invention, a nitro aspirin includes NCX 4040, and the like, but is not limited thereto.

In an example of the present invention, a COX-2 inhibitor includes Celecoxib, and the like, but is not limited thereto.

In an example of the present invention, an IDO inhibitor includes Indoximod, NLG919, and the like, but is not limited thereto.

In an example of the present invention, a PDE-5 inhibitor includes Tadalafil (Cialis), and the like, but is not limited thereto.

In an embodiment of the present invention, a solid cancer microenvironment immunosuppressive factor control material that a multi-domain vesicle contains may consist of combinations of at least two of the drugs described above.

In an embodiment of the present invention, the solid cancer microenvironment immunosuppressive factor control material may be an immunomodulatory material including a multi-domain vesicle capable of improving therapeutic efficacy, in which natural killer cells and T cells that have a therapeutic ability to find and directly kill cancer cells present in the body effectively survive in the body.

An example of the present invention may provide a composition based on a multi-domain vesicle including antibodies serving to suppress an immune checkpoint (PD-1, PDL-1 CTLA-4, LAG-3, TIM-3, and CEACAM1) by a T cell activation method through direct binding in a solid cancer microenvironment.

In an example of the present invention, an anti-CTLA-4 antibody includes Ipilimumab, and the like, but is not limited thereto.

In an example of the present invention, an anti-PDl-antibody includes Nivolumab, and the like, but is not limited thereto.

In an example of the present invention, an anti-PDLl antibody includes Atezolizumab, and the like, but is not limited thereto.

In an example of the present invention, an anti-LAG-3 antibody includes BMS-986016, and the like, but is not limited thereto.

In an example of the present invention, an anti-TIM-3 antibody includes TSR-022, and the like, but is not limited thereto.

In an example of the present invention, an anti-CEACAMl antibody includes CM-24, and the like, but is not limited thereto.

An example of the present invention provides a composition based on a multi-domain vesicle including a co-activation factor (OX40, CD137, CD27, and CD40), and the like by a T cell activation method through direct binding in a solid cancer microenvironment.

In an example of the present invention, anti-OX40 includes RG7888, and the like, but is not limited thereto.

In an example of the present invention, anti-CD137 includes Urelumab, and the like, but is not limited thereto.

In an example of the present invention, anti-CD27 includes Varlilumab, and the like, but is not limited thereto.

In an example of the present invention, anti-CD40 includes BMS-986090, and the like, but is not limited thereto.

An example of the present invention provides a composition based on a multi-domain vesicle containing a drug capable of suppressing immunosuppressive inducing factors (Treg, MDSC, TAM, IDO, and PD-L1) by a T cell activation method through indirect binding in a solid cancer microenvironment.

An example of the present invention may provide a composition based on a multi-domain vesicle including an anticancer agent that increases the efficacy of immune cells by inducing immunogenic cell death through chemotherapy.

An example of the present invention provides a composition based on a multi-domain vesicle including a drug capable of killing cancer cells or controlling a tumor microenvironment through epigenetic machinery.

In the present invention, as an example of the epigenetic machinery, a DNA methyltransferase inhibitor (DNMTi) material includes one selected from 5-Azacytidine, 5-Aza-2-deoxycytidine, Decitabine, SGI-110, Zebularine, CP-4200, Cladribine, Fludarabine, Clofarabine, Procainamide, Procaine, Hydralazine, Disulfiram, RG108, Nanaomycin A, Genistein, Equol, Curcumin, EGCG, Resveratrol, Parthenolide, and the like, but is not limited thereto.

In the present invention, as an example of the epigenetic machinery, a histone deacetylase inhibitor (HDACi) material includes one selected from Vorinostat, Abexinostat, Suberoylanilide, Hydroxamic acid, Belinostat, Panobinostat, Romidepsin, Valproic acid, Entinostat, Givinostat, Resminostat, Quisinostat, Pracinostat, Dacinostat, Pyroxamide, CHR-3996, CBHA, Trichostatin A, Oxamflatin, MC1568, Tubacin, PCI-30451, Tacedinaline, Mocetinostat, Chidamide, BML-210, M344, Butyrate, Sodium butyrate, Trapoxin A, Apicidin, Nicotinamide, Splitomicin, EX-527, Dihydrocoumarin, Tenovin-D3, AGK2, AEM1, AEM2, Cambinol, Sirtinol, Salermide, Tenovin-6, TMP-269, Psammaplin A, Nexturastat A, RGFP966, and the like, but is not limited thereto.

Hereinafter, the present invention will be described in more detail through Examples of the present invention, but the following Examples are exemplified to help understanding of the present invention, and the contents of the present invention are not limited to the following Examples.

### Example 1. Production and characterization of multi-domain vesicle including immunomodulatory material

In the Examples of the present invention, a multi-domain vesicle was produced as follows.

### 1-1. Production and characterization of squalene-based multi-domain vesicle (imMDV(SQ))

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the formed double emulsion was dispersed in a dichloromethane solution. The dichloromethane was removed using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained. Further, a control was also produced in the same manner as in the Example, except that squalene was not included.

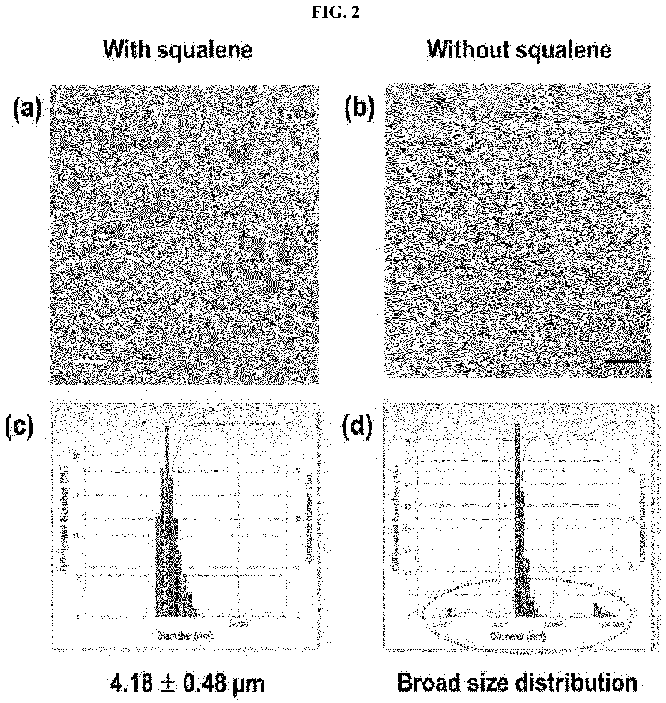

As a result of observing the structure of the multi-domain vesicle as described above using an optical microscope and dynamic light scattering (DLS, Otsuka Electronics Co., Ltd.), the multi-domain vesicle including squalene had a uniform size as compared to the control including no squalene, and exhibited a clear boundary even at the interface with the dispersion phase [FIGS. 2(A) and 2(B)]. In contrast, it could be seen that in the control including no squalene, irregular sizes and shapes were maintained [FIGS. 2(C) and 2(D)].

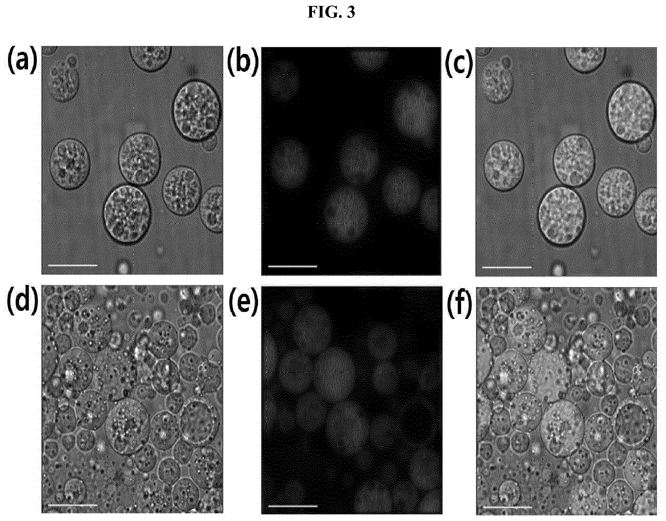

In addition, FIGS. 3(A) to (C) are optical microscope images of a multi-domain vesicle including squalene, and FIGS. 3(D) to (F) are optical microscope images of a multi-domain vesicle including no squalene. As illustrated in FIG. 3, it could be seen that the structure of the multi-domain vesicle could be clearly observed by solubilizing a rhodamine fluorescent dye in an oil phase solution, and the multi-domain vesicle including squalene exhibited a clear boundary point and was dispersed in an aqueous solution.

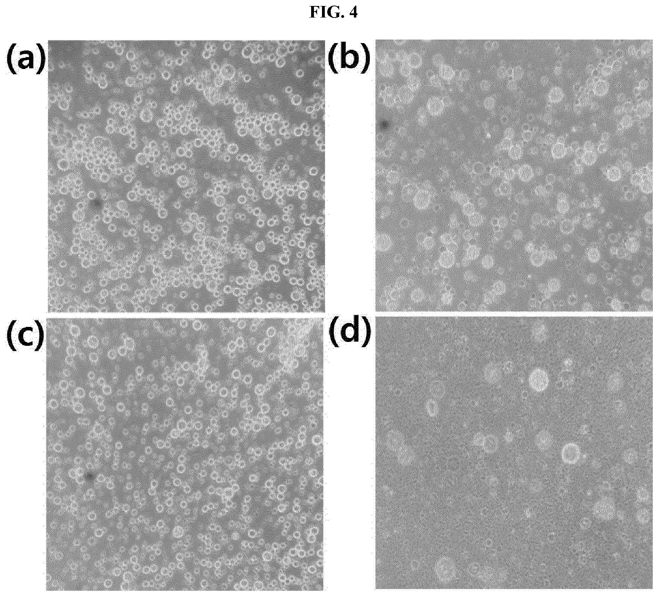

When a process of removing impurities to analyze the stability after the production or a centrifugation (about 2,500 rpm) process to classify the multi-domain vesicle according to the size was performed, it could be confirmed that the structure of the multi-domain vesicle including squalene [FIGS. 4(A) and 4(C)] formed a stable structure while minimally changing the shape before and after the centrifugation, but most of the structure in the control including no squalene was destroyed [FIGS. 4(B) and 4(D)].

### 1-2. Production and characterization of multi-domain vesicle (imMDV-1:imMDV(MPLA)) including squalene-based MPLA

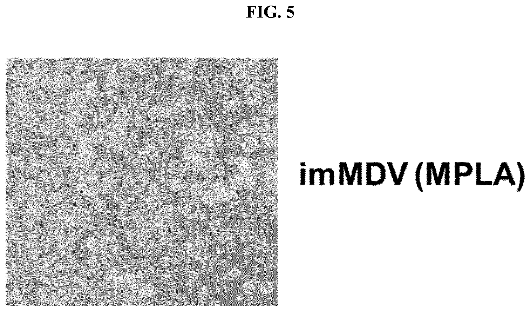

An oil phase solution was produced by dissolving DOPC (10 mg), cholesterol (8 mg), monophosphoryl lipid A [MPLA, 10 mg, Avanti Polar Lipids, USA], squalene (12 mg), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the formed double emulsion was dispersed in a dichloromethane solution. The dichloromethane was removed using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained. FIG. 5 is an optical image of a multi-domain vesicle (imMDV-1:imMDV(MPLA)) including squalene-based MPLA.

### Evaluation of immune cell activation effect of produced multi-domain vesicle

Effects of imMDV(SQ) and imMDV(MPLA) samples produced in Examples 1-1 and 1-2 on the activation of bone marrow-derived dendritic cells (BMDCs) and bone marrow-derived macrophages (BMMs) were analyzed from the secretion amounts of pro-inflammatory cytokines (TNF-α, IL-6, and IL-12) using an ELISA experimental method.

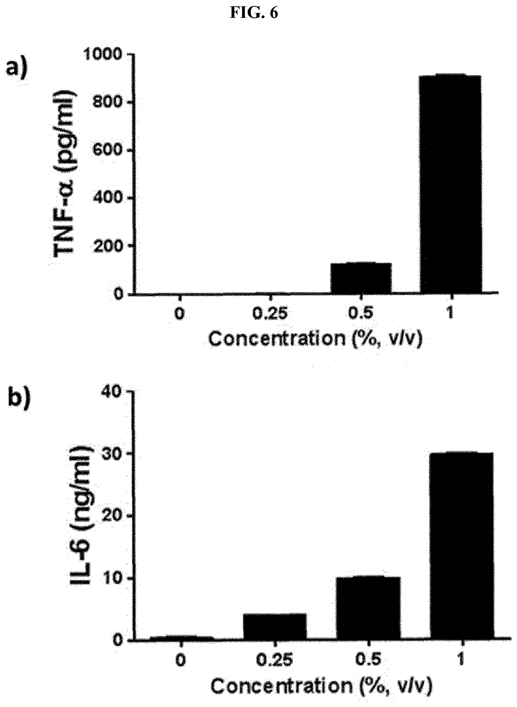

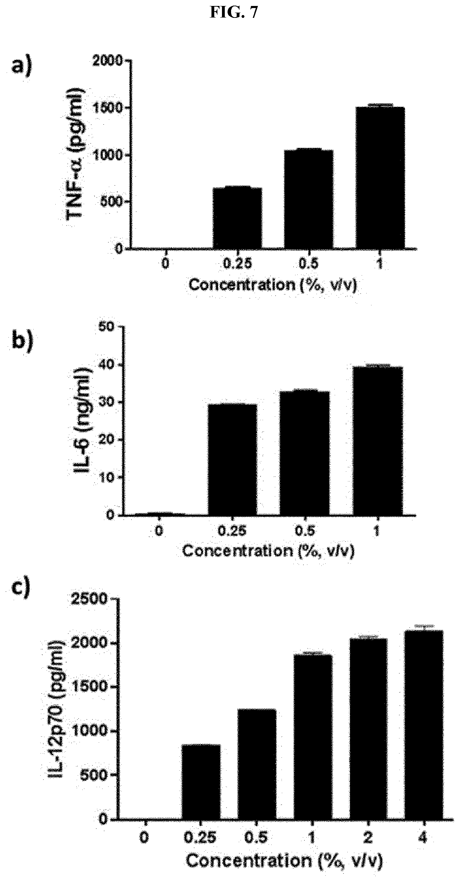

In FIG. 6, it was confirmed that when imMDV(SQ) was treated, the secretion of TNF-α and IL-6 was increased in proportion to the treated concentration, and further in FIG. 7, it could be confirmed that even when imMDV(MPLA) was treated, the secretion of TNF-α, IL-6, and IL-12 was increased in proportion to the concentration of the treated multi-domain vesicle.

### Production of multi-domain vesicle (imMDV) including squalene-based antigen (ovalbumin) and characterization of release behavior

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose) including ovalbumin (5 mg, Sigma-Aldrich, USA) for 10 minutes using a homogenizer (20,000 X g). Thereafter, the mixed solution was vortexed in 3 mL of an external aqueous phase (7.5% glucose, 40 mM lysine) for 10 seconds. Finally, the formed double emulsion was dispersed in a dichloromethane solution. The dichloromethane was removed using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained. Further, a control was also produced in the same manner as in the Example, except that squalene was not included.

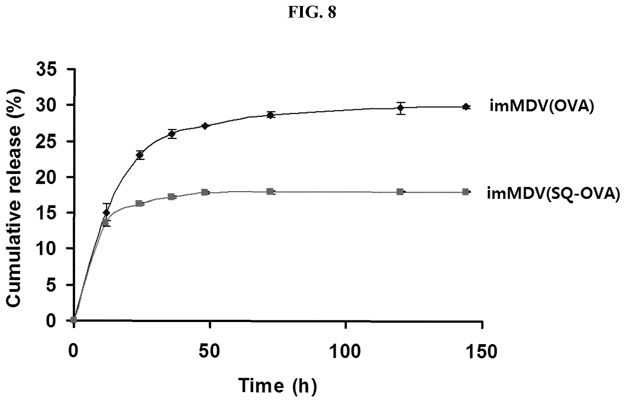

As a result of confirming the release behaviors of a multi-domain vesicle including an ovalbumin antigen and squalene (imMDV(SQ-OVA)) and a multi-domain vesicle including only an ovalbumin antigen (imMDV(OVA)), it could be confirmed that the ovalbumin antigen was slowly released from the multi-domain vesicle including squalene due to the delayed release behavior of the ovalbumin antigen [FIG. 8].

### 1-3. Production of multi-domain vesicle (imMDV-2) including squalene-based DDA

An oil phase solution was produced by dissolving DOPC (10 mg), cholesterol (8 mg), DDA (10 mg, Avanti Polar Lipids, USA), squalene (12 mg), and glycerol trioleate (12 mg) in chloroform (1 mL). Thereafter, the supernatant was removed after settling the oil phase solution with a centrifuge, and liposomes were obtained. The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-4. Production of multi-domain vesicle (imMDV-3) including squalene-based MPLA/TDB

An oil phase solution was produced by dissolving DOPC (10 mg), cholesterol (8 mg), MPLA (10 mg), squalene (12 mg), TDB (10 mg, Avanti Polar Lipids, USA), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-5. Production of multi-domain vesicle (imMDV-4) including squalene-based MPLA/DDA

An oil phase solution was produced by dissolving DOPC (10 mg), cholesterol (8 mg), MPLA (10 mg), DDA(10 mg), squalene (12 mg), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-6. Production of multi-domain vesicle (imMDV-5) including squalene-based MPLA/OS21

An oil phase solution was produced by dissolving DOPC (10 mg), cholesterol (8 mg), MPLA (10 mg), QS21 (10 mg, Desert King, USA), squalene (12 mg), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-7. Production of multi-domain vesicle (imMDV-6) including squalene-based CpG

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose, 1 mg of CpG, Bioneer, Korea) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-8. Production of multi-domain vesicle (imMDV-7) including squalene-based Poly(I:C)

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase [5% sucrose, 1 mg of Poly(I:C) (Sigma-Aldrich, USA)] for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-9. Production of multi-domain vesicle (imMDV-8) including squalene-based resquimod

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose, 5 mg of resquimod (Sigma-Aldrich, USA)) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-10. Production of multi-domain vesicle (imMDV-9) including squalene-based imiquimod and confirmation of immune response induction effect

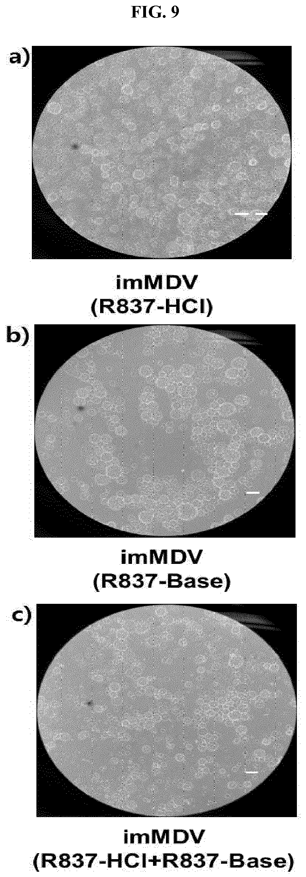

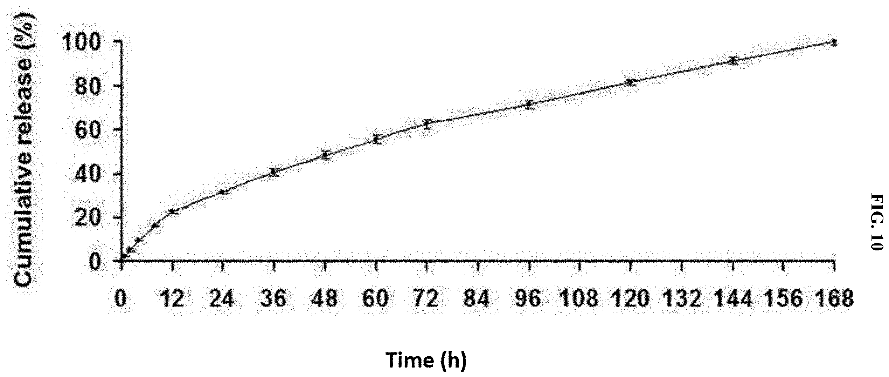

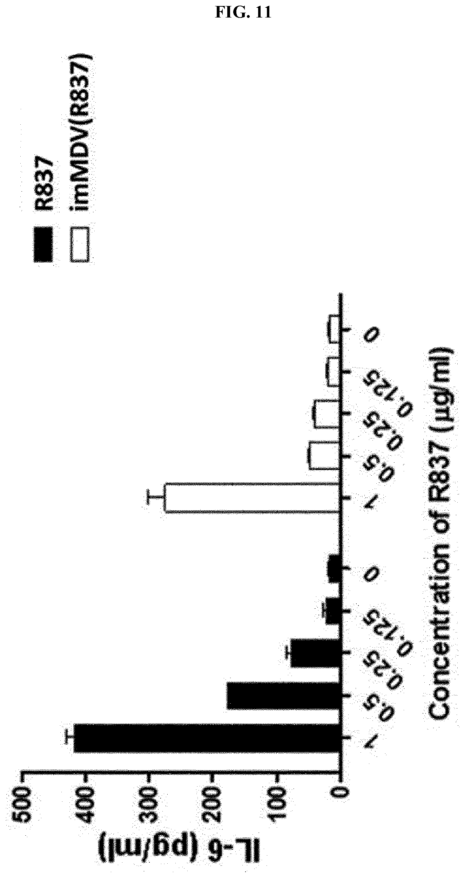

An oil phase solution was produced by dissolving DOPC (10 mg), cholesterol (8 mg), squalene (12 mg), oleic acid (2 mg, Sigma-Aldrich, USA), imiquimod (base form, Sigma-Aldrich, USA)(5 mg), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained. Imiquimod in the HCl form to be dissolved in an aqueous solution was produced from imiquimod in the base form through the process as described below. 400 g of imiquimod was dissolved in a mixed solution of 2000 ml of distilled water and 900 ml of n-butanol (or 1-butanol). 150 ml of a 37% HCl solution was simultaneously further added thereto while stirring. After the solution was stirred in a range of 60 to 65°C until the imiquimod was completely dissolved, the solution was cooled to 20 to 25°C, and then maintained for about 30 minutes. When the remaining solution was dried in a dryer at room temperature, HCl imiquimod may be obtained. After the thus-produced HCl-imiquimod was dissolved in the internal aqueous phase during the production process of imMDV, the imMDV was produced by subjecting the resulting solution to the same process. FIG. 9 illustrates optical microscope images of multi-domain vesicles into which imiquimod in the HCl form is loaded, imiquimod in the base form is loaded, and both forms of imiquimod are simultaneously loaded. Among the obtained multi-domain vesicles, the release behavior of imiquimod in (imMDV(R837-HCl)) was analyzed at 37°C using a transwell. The amount of drug released was quantified using a UV-Vis spectrometer (FIG. 10). As illustrated in FIG. 10, about 70% of the loaded drug was released over 8 days. Further, when bone marrow-derived dendritic cells (BMDCs) are treated with the imMDV(R837-HCl) sample, the secretion amount of a typical pro-inflammatory cytokine IL-6 associated with a Th1 immune response was analyzed using an ELISA experimental method. As illustrated in FIG. 11, it was confirmed that the secretion of IL-6 was increased in proportion to the treated concentration, and it can be seen that R837-HCl encapsulated in the multi-domain vesicle is released to activate immune cells by confirming that a behavior similar to that of R837-HCl used as a control is exhibited.

Effects of the multi-domain vesicles [imMDV(R837-HCl), imMDV(R837-base), and imMDV[R837-HCl:R837-base] including imiquimod produced in the Example on the formation of antibodies against an ovalbumin model antigen were verified through a mouse experiment (C57BL/6, 6- to 7-week-old females). It was determined by an enzyme linked immunosorbent assay (ELISA) method that a humoral immune response was increased as 50 µg of the multi-domain vesicle was injected into the mice.

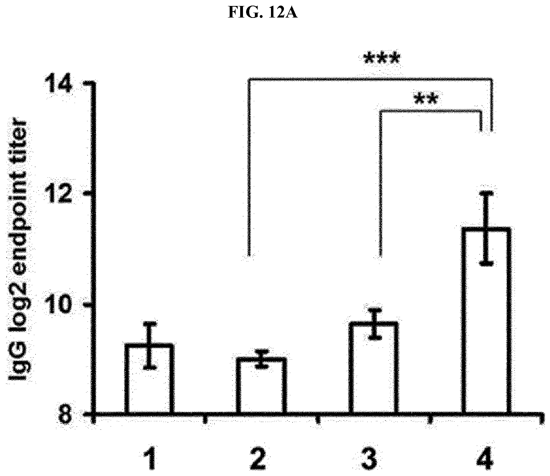

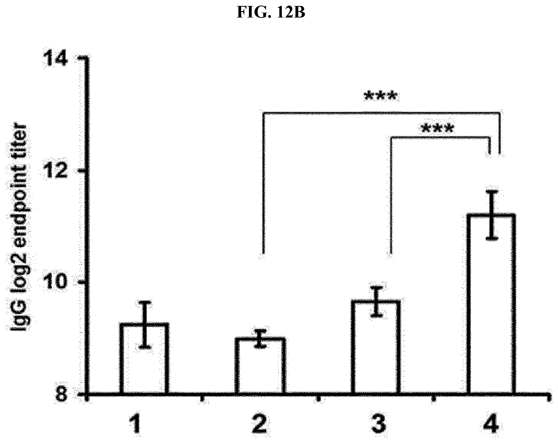

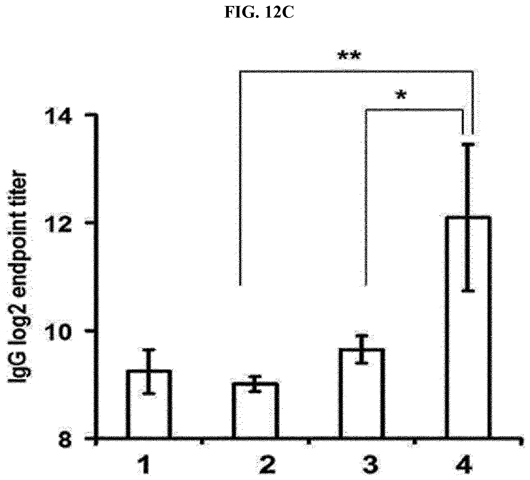

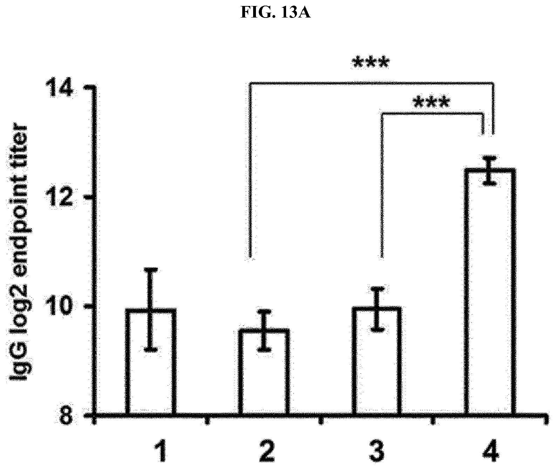

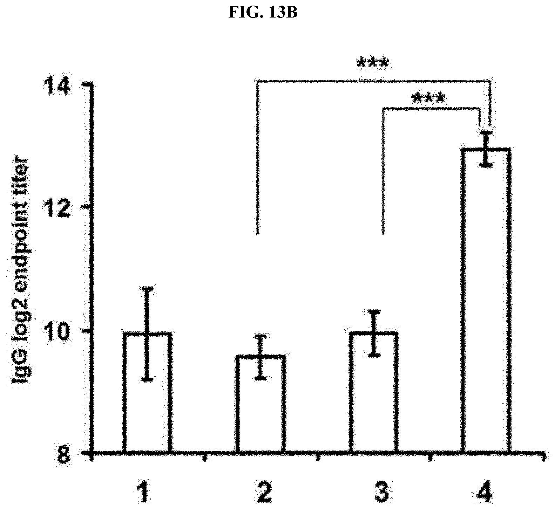

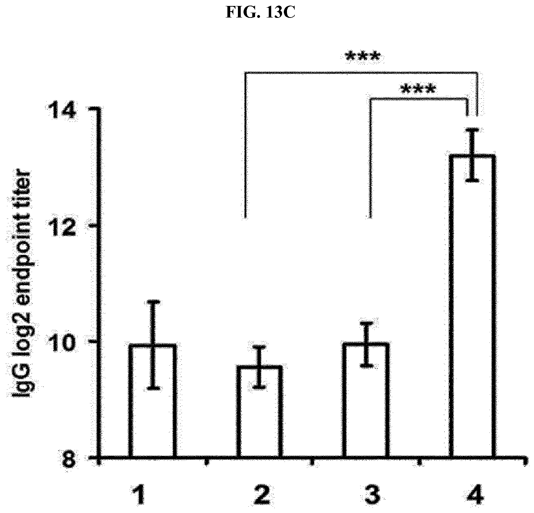

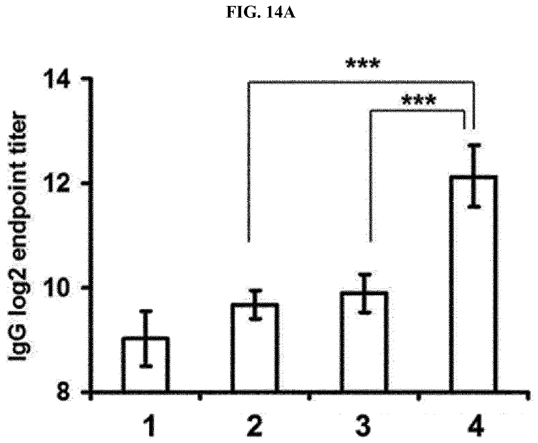

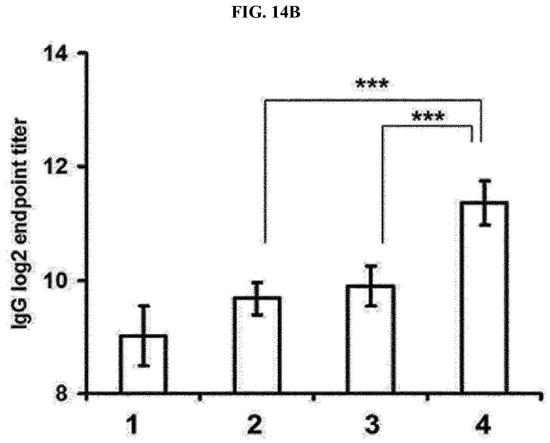

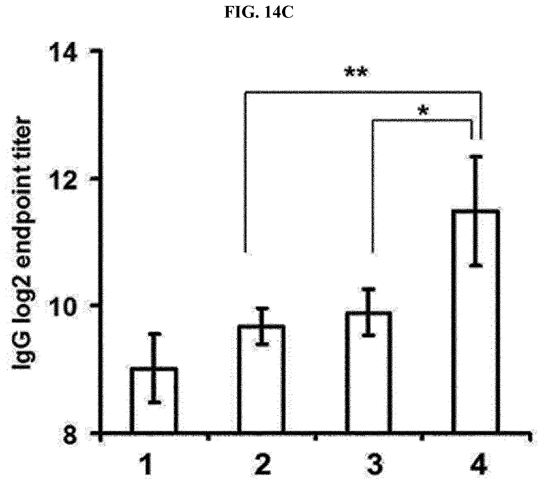

As can be seen in FIGS. 12A, 12B, and 12C, it can be confirmed that the humoral immune effect (IgG, 1 week after injection) against the ovalbumin (OVA) model antigen is remarkably increased in the experimental groups to which the multi-domain vesicle samples (12a:imMDV(R837-HCl) sample, 12b: imMDV(R837-base) sample, and 12c: imMDV[R837-HCl:R837-base (1:1) sample]) into which imiquimod is loaded are administered. Further, it can be confirmed that the humoral immune effect increased in this manner is sustained even after 3 weeks (FIGS. 13A, 13B, and 13C) and 5 weeks (FIGS. 14A, 14B, and 14C) after the injection have passed.

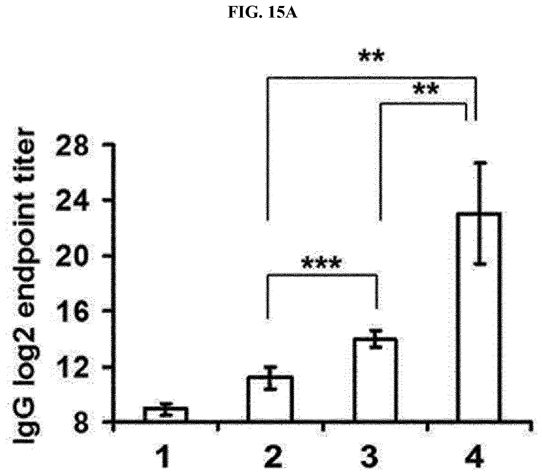

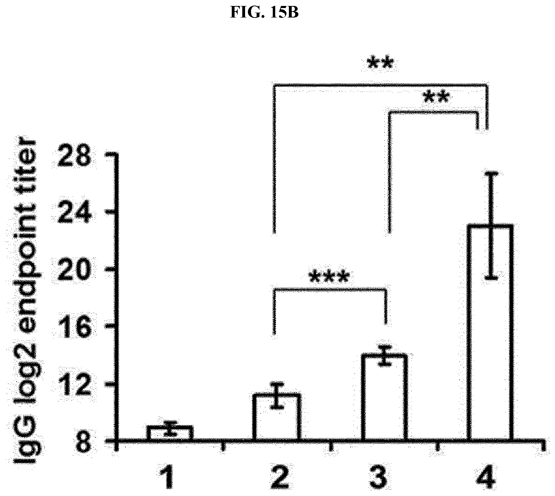

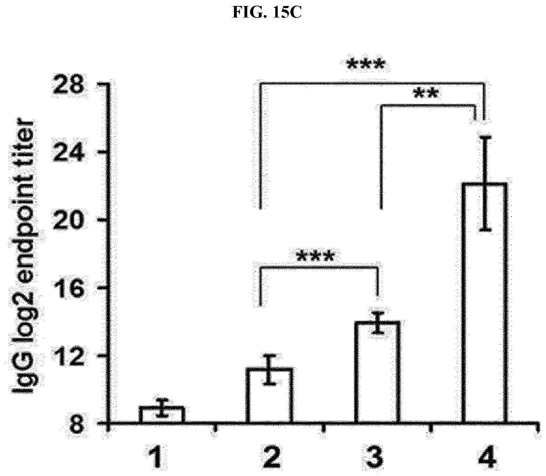

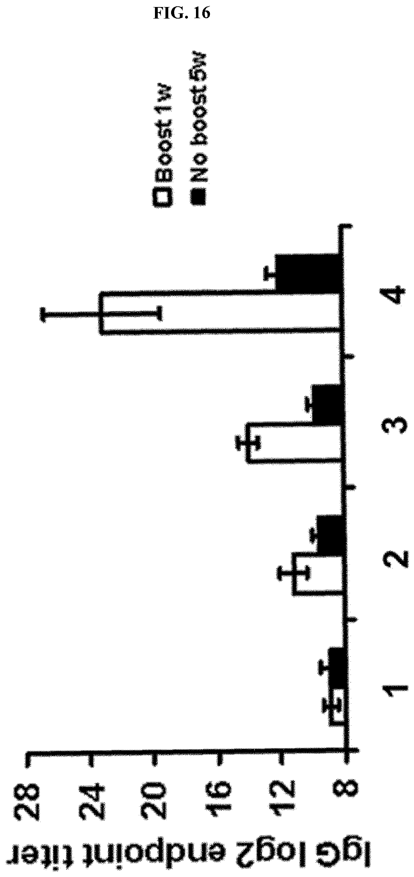

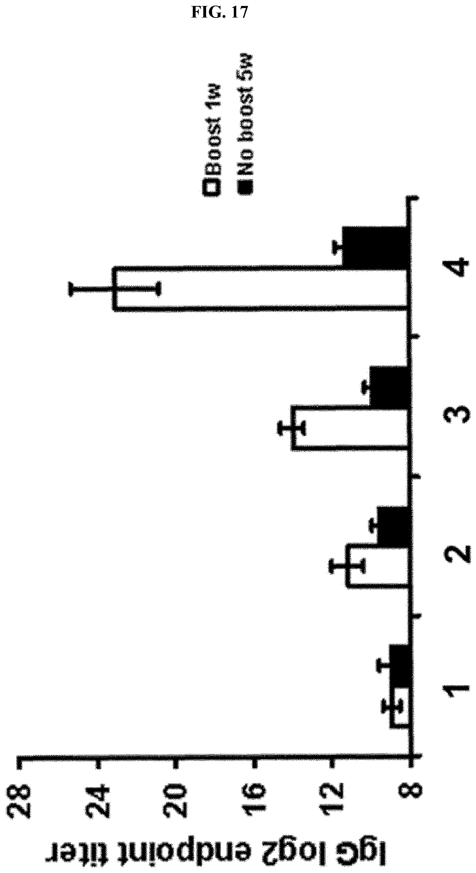

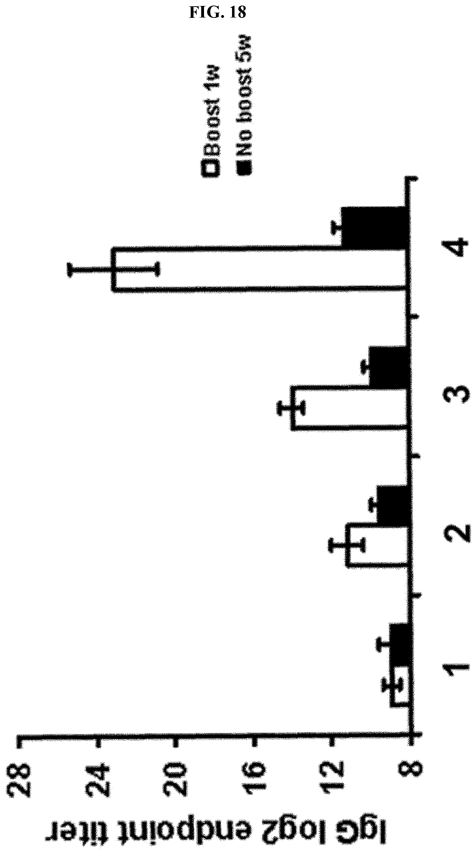

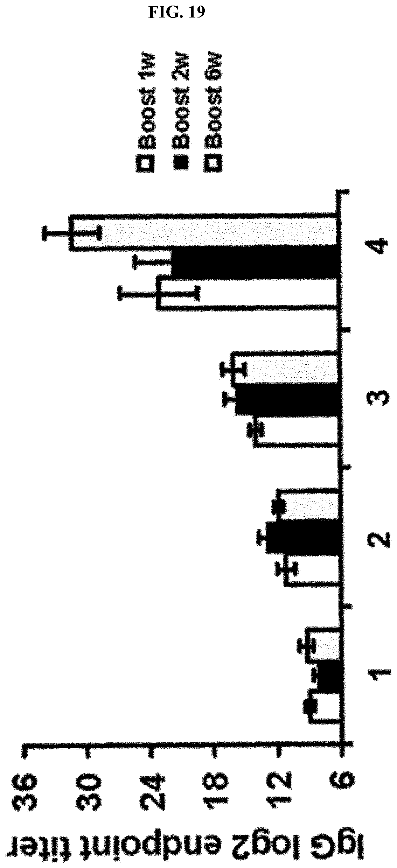

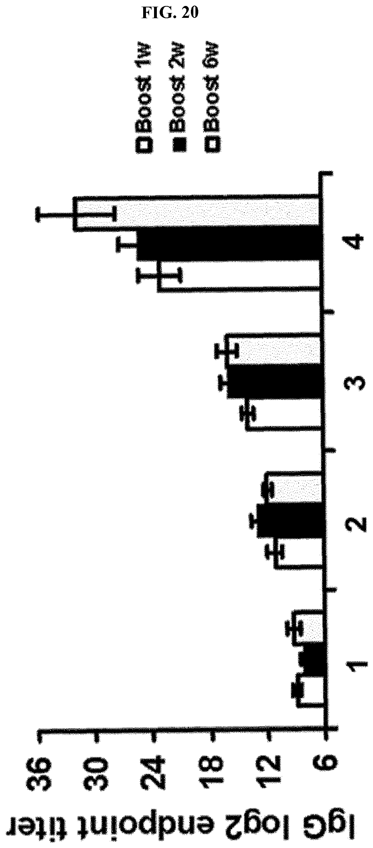

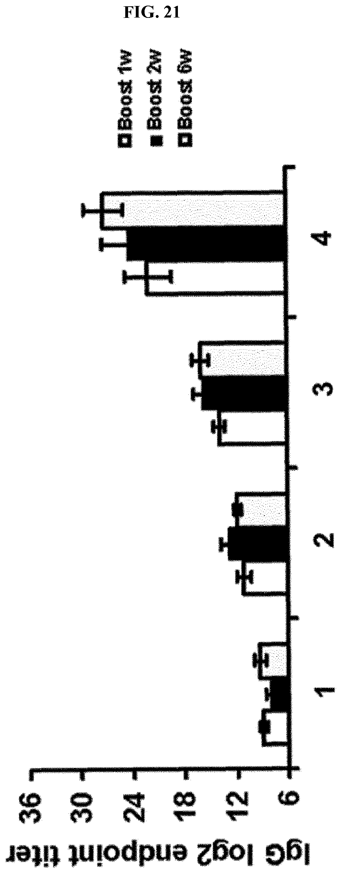

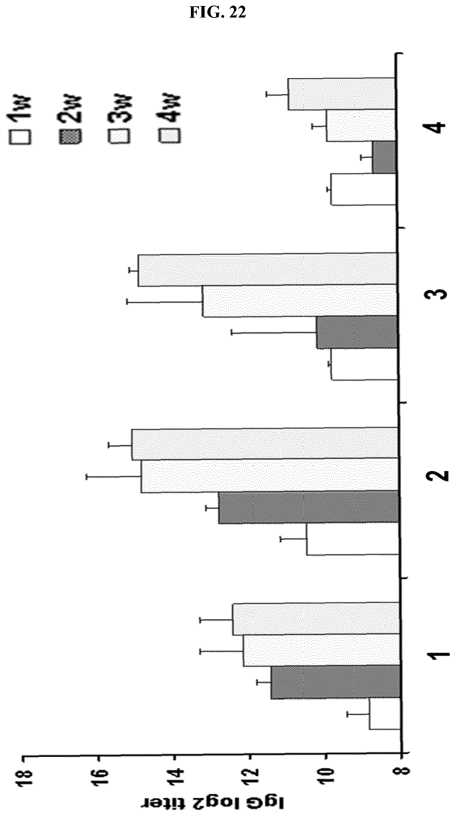

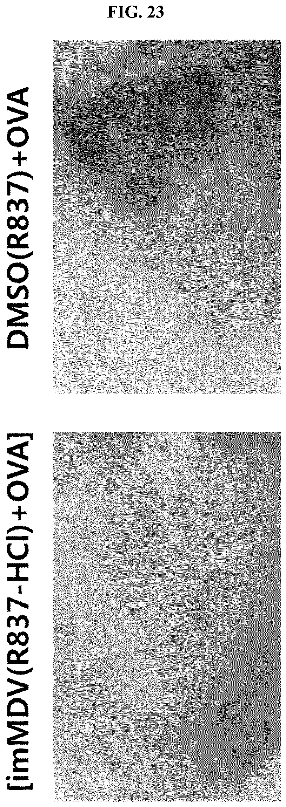

It can be seen that the humoral immune effect is remarkably increased when boosting is additionally performed once at the time point when five weeks after the first injection have passed (FIGS. 15A, 15B, 15C, 16, 17, and 18). It can be confirmed that the increased humoral immune effect is sustainably maintained even 1, 2, and 6 weeks after boosting at week 5 (FIGS. 19, 20, and 21). It can be confirmed that induction of immune enhancement and sustainability effects by the multi-domain vesicle-based adjuvant are excellent even when compared with those of an adjuvant in the oil form (DMSO(R837)) used at the clinical stage (FIG. 22). Above all, the greatest advantage is that the inflammation phenomenon occurring when an adjuvant in the oil form (DMSO(R837)) is administered does not occur at all when a multi-domain vesicle-based adjuvant (imMDV(R837-HCl)) is used (FIG. 23).

### 1-11. Production of multi-domain vesicle (imMDV-10) including squalene-based STING (cyclic DNA)

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose, 1 mg of STING (InvivoGen, USA)) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-12. Production of multi-domain vesicle (imMDV-11) including squalene-based MPLA/CpG

An oil phase solution was produced by dissolving DOPC (10 mg), cholesterol (8 mg), squalene (12 mg), MPLA (10 mg), and glycerol trioleate (12 mg) in chloroform (1 mL). The produced oil phase solution was dispersed in 1 mL of an internal aqueous phase (5% sucrose, 1 mg of CpG) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-13. Production of multi-domain vesicle (imMDV-12) including squalene-based MPLA and Poly(I:C)

An oil phase solution was produced by dissolving DOPC (10 mg), cholesterol (8 mg), squalene (12 mg), MPLA (10 mg), and glycerol trioleate (12 mg) in chloroform (1 mL). The produced oil phase solution was dispersed in 1 mL of an internal aqueous phase [5% sucrose, 1 mg of Poly(I:C)] for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-14. Production of multi-domain vesicle (imMDV-13) including squalene-based CpG/Poly(I:C)

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase [5% sucrose, 1 mg of CpG, and 1 mg of Poly(I:C)] for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-15. Production of multi-domain vesicle (imMDV-14) including castor oil-based MPLA

An oil phase solution was produced by dissolving DOPC (10 mg), cholesterol (8 mg), MPLA (10 mg), castor oil (12 mg, Sigma-Aldrich, USA), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### 1-16. Production of multi-domain vesicle (imMDV-15) including mineral oil-based MPLA

An oil phase solution was produced by dissolving DOPC (10 mg), cholesterol (8 mg), MPLA (10 mg), mineral oil (12 mg, Sigma-Aldrich, USA), and glycerol trioleate (12 mg) in chloroform (1 mL). The produced oil phase solution was dispersed in 1 mL of an internal aqueous phase (5% sucrose) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and liposomes were obtained.

### Example 2. Evaluation of immune enhancement efficacy of multi-domain vesicle against viral antigen

In order to investigate a specific immune effect against avian influenza viruses of multi-domain vesicle samples including the immune function modulatory material produced in Example 1, effects of B cells associated with particularly the production of antibodies during an antibody-specific immune response on the humoral immune response were investigated. First, female BALB/c and C57BL/6 mice (5 to 6 weeks old) were purchased from KOATECH (Korea, Pyeongtaek). All experiments using mice were performed in accordance with the Korean NIH guidelines for the care and use of laboratory research animals.

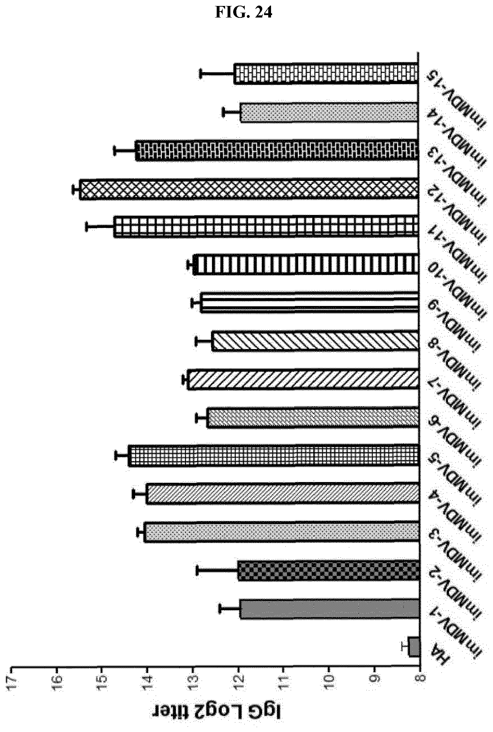

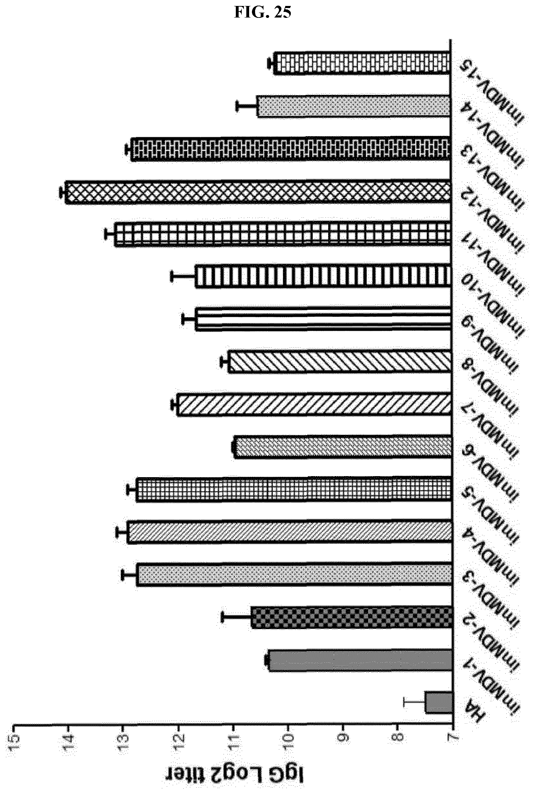

Mouse sera were collected 2 weeks (FIG. 24) and 4 weeks (FIG. 25) after the first intramuscular injection, and the antibody titer against the HA protein in the serum was measured by an enzyme linked immunosorbent assay (ELISA) method. In the ELISA method, a plate coated with the HA protein was blocked using PBS/3% bovine serum albumin (BSA), and then the control experimental group serum was incubated at various serial dilutions. Thereafter, mouse IgG to which horseradish peroxidase was attached was added thereto. All the incubations were performed at 37°C for 1 hour, and the control experimental group serum was washed 3 times with PBS/0.05% Tween 20 after each step mentioned above. After a reaction was developed by adding 100 µL of tetramethylbenzidine (TMB, BD Biosciences, USA) as a substrate thereto, the absorbance at 450 nm was measured by an ELISA reader, and the results are shown in FIGS. 24 and 25.

### Example 3. Evaluation of immune enhancement efficacy of multi-domain vesicle against cancer antigen

### 3-1: Confirmation of OVA-specific antibody production of multi-domain vesicle

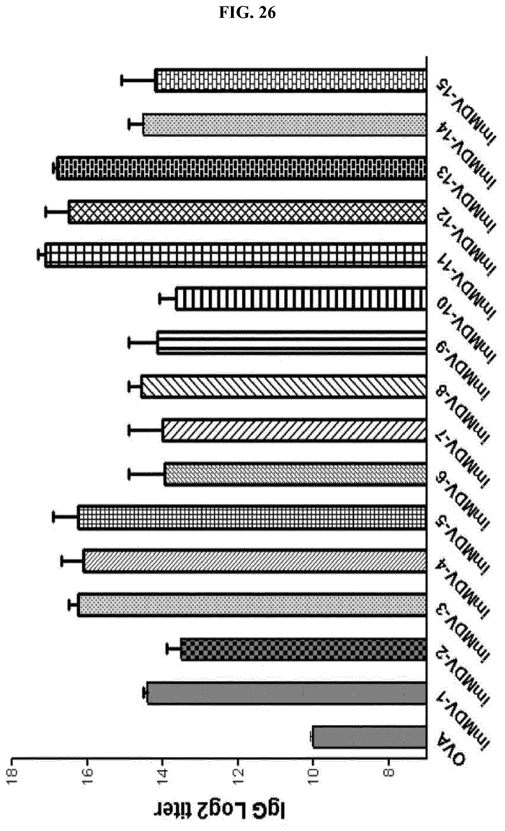

Cancer prevention vaccine effects of the multi-domain vesicle including the immune function modulatory material produced in Example 1 were verified through a mouse experiment (C57BL/6, 6- to 7-week-old females). It was determined by an enzyme linked immunosorbent assay (ELISA) method that a humoral immune response was increased as 50 µg of an immunomodulatory material (cancer prevention vaccine) including the multi-domain vesicle was injected into the mice, and the results are shown in FIG. 26 (measurement of the amount of IgG produced). The humoral immune response was confirmed by performing an ophthalmic blood sampling in mice after vaccination to compare the amount of immunoglobulin G (IgG) produced with that of the control group.

### 3-2: Confirmation of specific cell-mediated T cell response by multi-domain vesicle

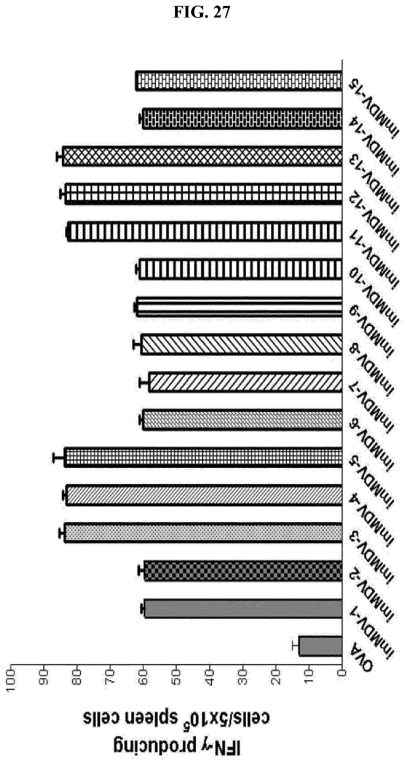

A cellular immune response of T cells in a mouse spleen by a multi-domain vesicle including an immune function modulatory material was investigated. Three mice were selected from OVA and OVA-multi-domain vesicle groups among the mice inoculated in Example 3-1, and after two weeks, the spleen was removed from each mouse, and then the spleen tissue was transferred to a sterilized petri dish, the spleen was ground using a cell strainer, and cells were isolated from the tissue epithelium. After all contents in the petri dish were transferred to a 50-mL tube and the tube was filled with an RPMI medium, the tube was centrifuged at 1,500 rpm for 5 minutes, and then 5 mL of a red blood cell lysing buffer (Sigma Aldrich, Germany) was added to a pellet from which a supernatant had been removed, and the red blood cells were lysed by allowing the pellet to stand in a water bath at 30°C for 5 minutes to 10 minutes. Cells included in the tube were washed with PBS, and then suspended in an RPMI medium to isolate splenocytes. The isolated splenocytes were spread on a 96-well at 5 x 10⁵ cells/100 µL on a plate coated with IFN-gamma and treated with an MHC class I-restricted OVA peptide at a concentration of 5 µg/mL for 48 hours. Thereafter, IFN-gamma to which horseradish peroxidase was attached was added thereto. After a reaction was developed by adding 100 µL of 3-amino-9-ethyl-carbazole (ACE, BD Biosciences, USA) as a substrate thereto, a measurement was made by an enzyme-linked immunospot (ELISPOT) method (FIG. 27).

### Example 4. Production of multi-domain vesicle loaded with drug for modulating solid cancer microenvironment

### 4-1. Production of multi-domain vesicle containing material for removing MDSCs

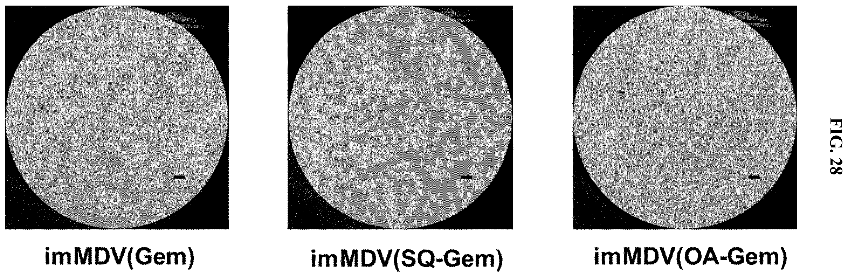

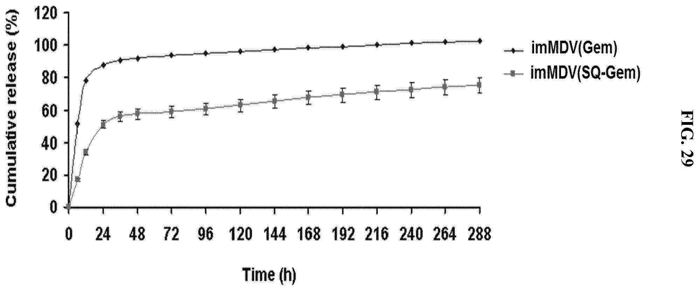

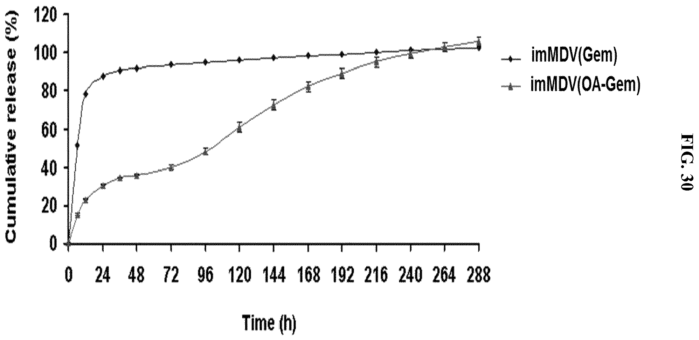

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose, Gemcitabine (Gemzar® (Eli Lilly and Company, Indianapolis, Ind., USA)), 5mg) for 10 minutes using a homogenizer (20,000 X g). Thereafter, the mixed solution was vortexed in 3 mL of an external aqueous phase (7.5% glucose, 40 mM lysine) for 10 seconds. Finally, the chloroform was removed from the formed double emulsion using a vacuum evaporator, and the residual solvent was removed by increasing the temperature to 37°C. The supernatant was removed after precipitating the solvent-free multi-domain vesicle dispersion at low temperature or settling the dispersion with a centrifuge, and a multi-domain vesicle (imMDV(SQ-Gem)) was obtained. A multi-domain vesicle (imMDV(OA-Gem)) loaded with gemcitabine while including oleic acid oil instead of squalene fluid oil and a multi-domain vesicle (imMDV(Gem)) loaded with gemcitabine while including no squalene may be produced by the same process as described above. FIG. 28 illustrates optical microscope images of the three samples thus produced. It can be confirmed that the loaded gemcitabine in the multi-domain vesicle including squalene is slowly released, whereas most of the loaded drug is released from the multi-domain vesicle including no squalene within 24 hours (FIG. 29). It can be confirmed that when oleic acid vegetable oil is used instead of an animal oil such as squalene, the sustained release behavior of loaded gemcitabine exhibits a plateau shape for 24 to 72 hours, and then exhibits a linear behavior after 72 hours. This implies that the release behavior of the loaded drug may be modulated using a fluid oil (FIG. 30).

### 4-2. Production of multi-domain vesicle including drug inducing immunogenic cell death

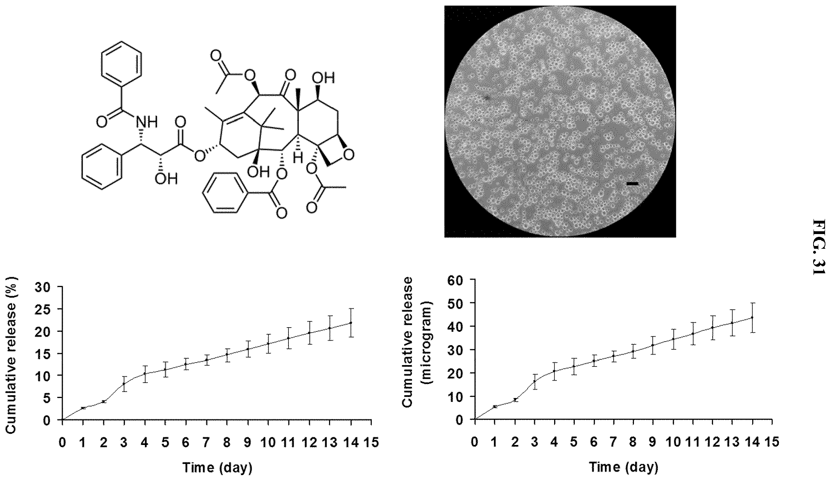

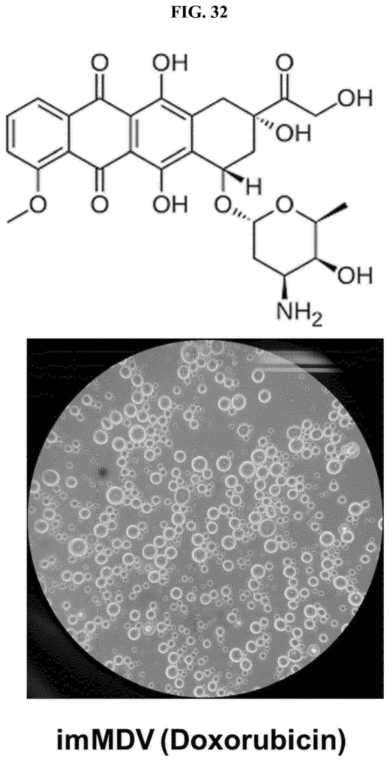

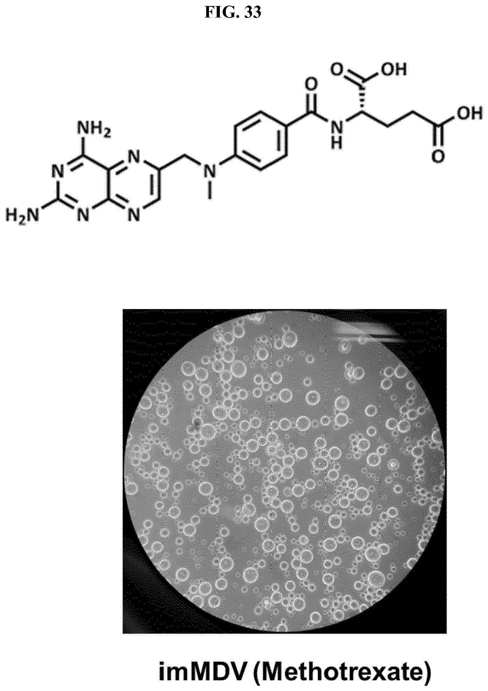

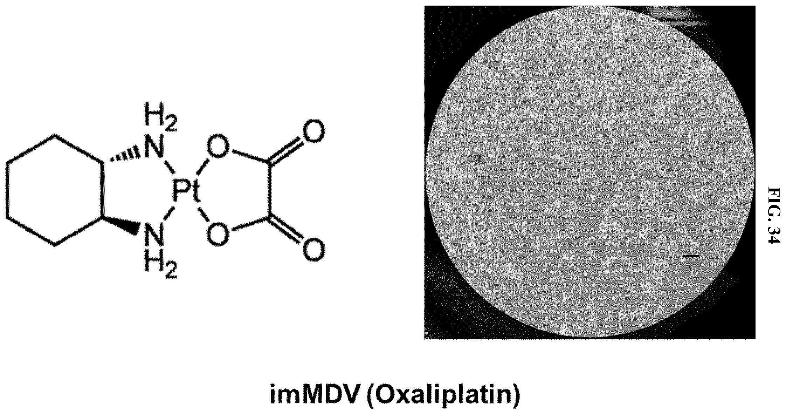

By inducing the death of cancer cells, paclitaxel, doxorubicin, methotrexate, and oxaliplatin were selected from among anticancer agents which serve to enable antigen-presenting cells to effectively recognize a cancer agent and multi-domain vesicles loaded with these drugs were produced. The multi-domain vesicles were produced using the same method as in Example 4-1, but imMDV (paclitaxel) (FIG. 31) was used by adding the paclitaxel drug to an oil phase solution, and multi-domain vesicles such as imMDV (doxorubicin) (FIG. 32), imMDV (methotrexate) (FIG. 33), and imMDV (oxaliplatin) (FIG. 34) were produced by adding each drug to an internal aqueous phase. As can be seen from FIG. 31, it could be confirmed that the loaded drug was slowly released over 2 weeks.

### 4-3. Production of multi-domain vesicle containing material for removing Treg cells

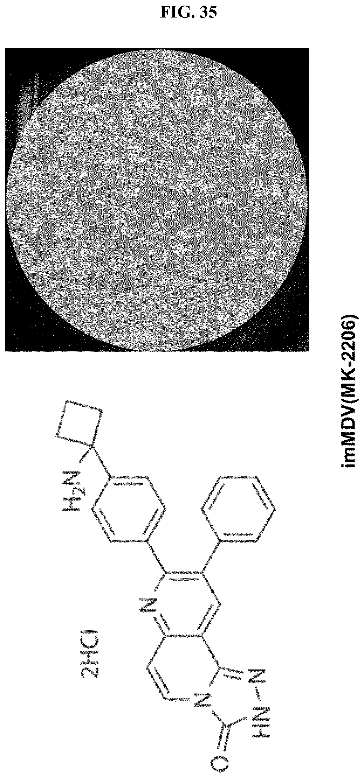

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose, (Imatinib: Gleevec® (Novartis Pharmaceuticals Corp, East Hanover, NJ, USA)) 5mg) in which MK-2206 (an Akt inhibitor, SelleckChem, 5mg) was dispersed for 10 minutes using a homogenizer (20,000 X g). Through the same subsequent processes as in Example 4-1, a multi-domain vesicle was produced (FIG. 35).

### 4-4. Production of multi-domain vesicle containing drug capable of controlling PI3K signaling

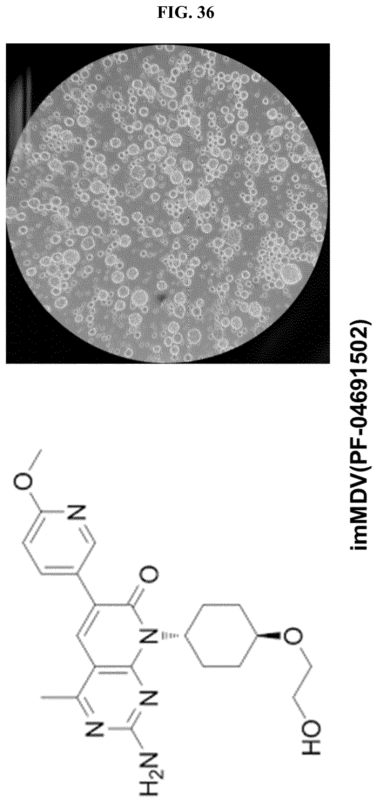

An oil phase solution was produced by dissolving DOPC (10 mg), cholesterol (8 mg), squalene (12 mg), and glycerol trioleate (12 mg) in chloroform (1 mL). The produced oil phase solution was dispersed in 1 mL of an internal aqueous phase (5% sucrose, (Imatinib: Gleevec® (Novartis Pharmaceuticals Corp, East Hanover, NJ, USA)) 5mg) in which PF-04691502(PI3K inhibitor, SelleckChem, 5mg) was dispersed for 10 minutes using a homogenizer (20,000 X g). Through the same subsequent processes as in Example 4-1, a multi-domain vesicle was produced (FIG. 36).

### 4-5. Production of multi-domain vesicle including drug capable of controlling epigenetic machinery

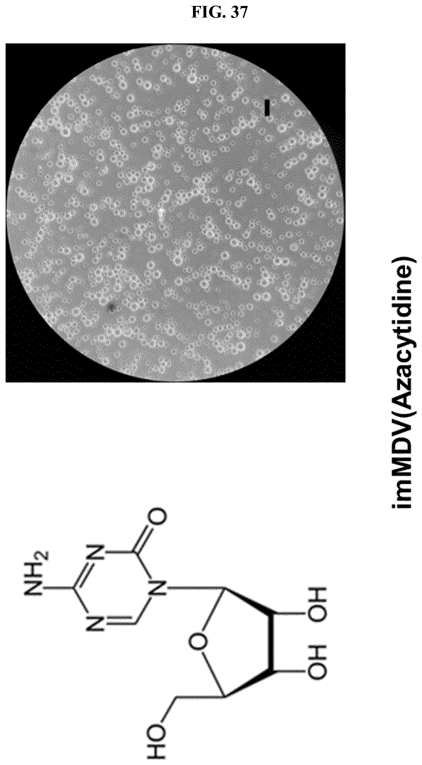

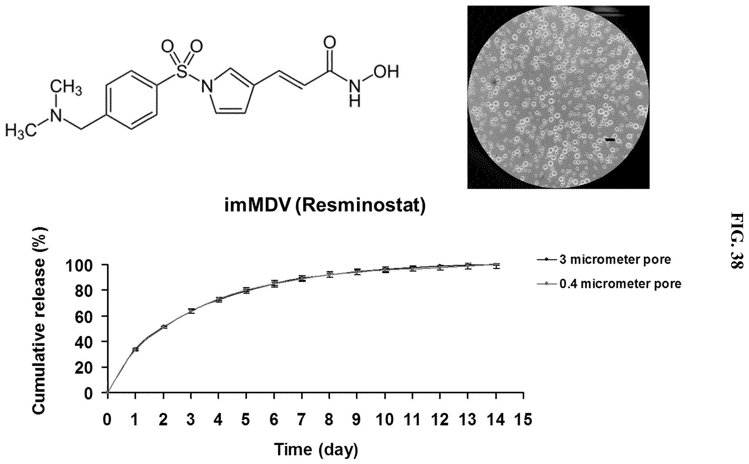

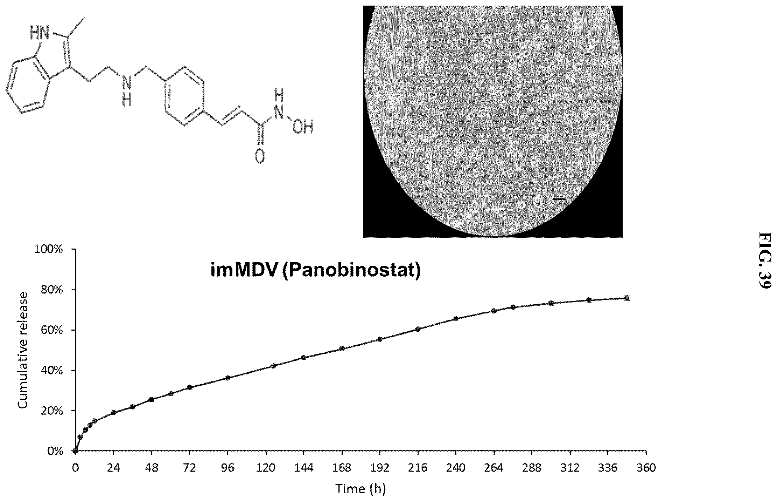

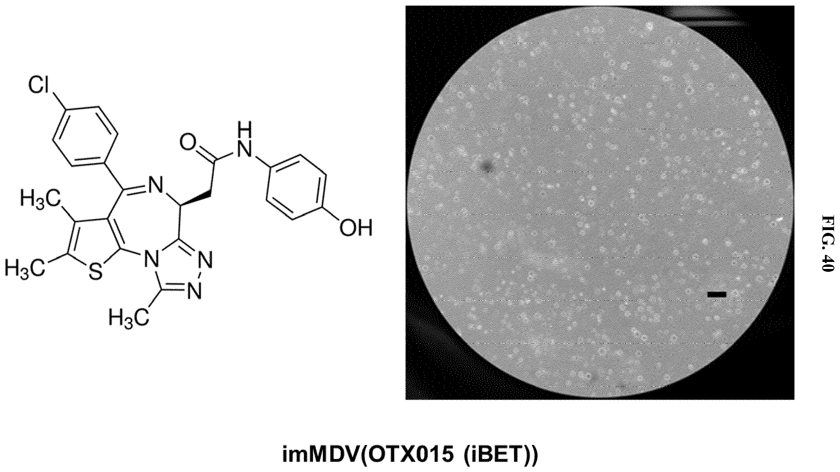

Among drugs capable of inducing the epigenetic machinery, Azacytidine, Resminostat, Panobinostat, and OTX015(iBET) were selected and multi-domain vesicles loaded with these drugs were produced. Specifically, multi-domain vesicles such as imMDV(Azacytidine) (FIG. 37), imMDV(Resmonostat) (FIG. 38), imMDV(Panobinostat) (FIG. 39), and imMDV(OTX015(iBET)) (FIG. 40) were produced by using the same method as in Example 4-1 to add each drug to the internal aqueous phase. As can be seen from FIGS. 38 and 39, it could be confirmed that the loaded drug was slowly released over 2 weeks.

### 4-6. Production of multi-domain vesicle containing material for removing TAM cells

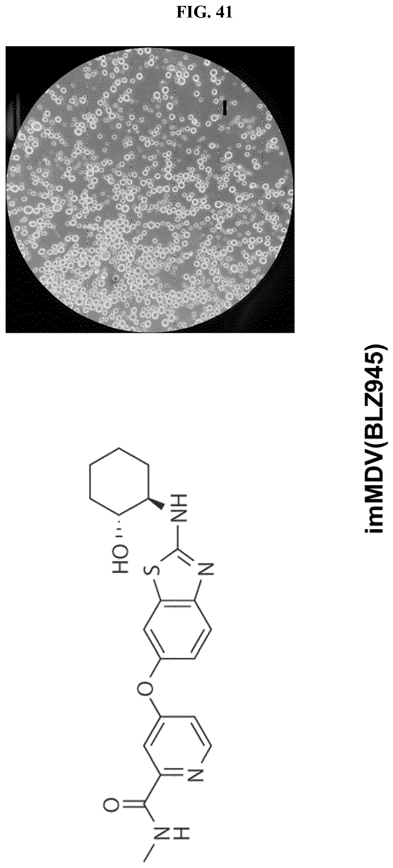

The same process as in Example 4-1 was used, but BLZ945 (CSF-1R kinase inhibitor) which is a drug capable of removing TAM cells was dissolved in an oil phase, and then a multi-domain vesicle was produced (FIG. 41).

### 4-7. Production of multi-domain vesicle containing tumor immunosuppressive cytokine inhibitor

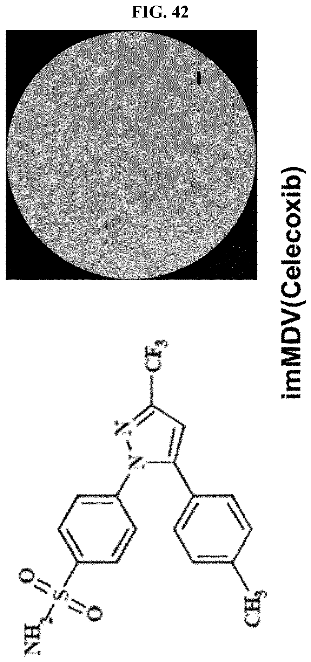

After 5 mg of the tumor immunosuppressive cytokine inhibitor drug (Celecoxib, Sigma-Aldrich) was dissolved in an oil phase in Example 4-1, a multi-domain vesicle was produced (FIG. 42).

### Example 5. Production of multi-domain vesicle in which solid cancer microenvironment immunomodulatory materials are combined

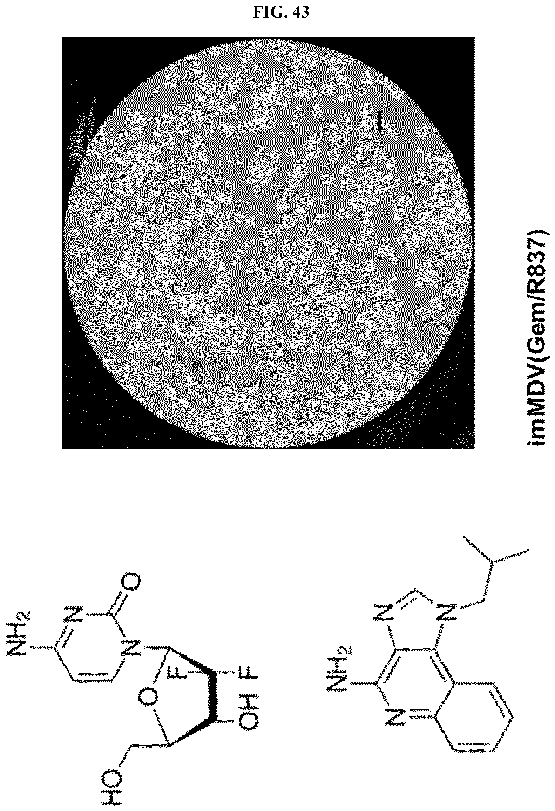

As an example of the production of a multi-domain vesicle in which materials modulating immune function in a solid cancer microenvironment were combined, a multi-domain vesicle (imMDV(GEM/R837)) having a stable structure while simultaneously containing gemcitabine (Example 4-1) capable of killing MDSCs and cancer cells and imiquimod (Example 1-9) which is a toll-like receptor serving to activate immune cells was produced (FIG. 43).

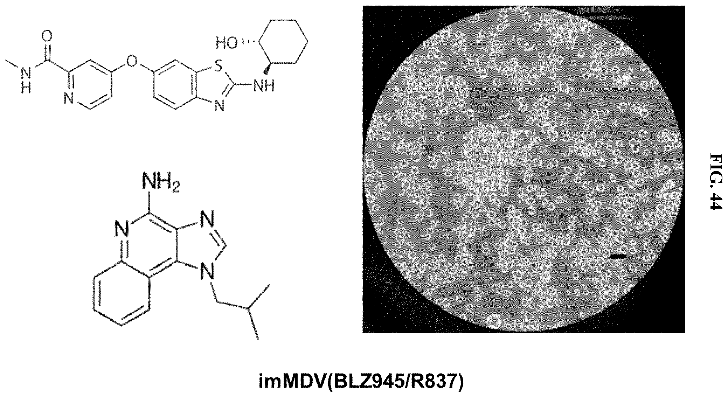

Further, a multi-domain vesicle (imMDV(BLZ945/R837)) having a stable structure while simultaneously containing BLZ945 (Example 4-6) which is a drug capable of removing TAM cells and imiquimod (Example 1-9) which is a toll-like receptor serving to activate immune cells was produced (FIG. 44).

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described Examples are illustrative only in all aspects and are not restrictive. For example, each constituent element which is described as a singular form may be implemented in a distributed form, and similarly, constituent elements which are described as being distributed may be implemented in a combined form. The scope of the present invention is represented by the claims to be described below rather than the detailed description, and it should be interpreted that the meaning and scope of the claims and all the changes or modified forms derived from the equivalent concepts thereto fall within the scope of the present invention.

### [Industrial Applicability]

The multi-domain vesicle according to the present invention may increase an effective duration time of an immunomodulatory material by simultaneously loading various immunomodulatory materials having hydrophilic properties inside liposomes and a lipophilic immunomodulation material onto the membrane of liposomes and/or the outer wall of the vesicle. Further, the method for producing a multi-domain vesicle according to the present invention has advantages in that the stability and storage stability in the production process of the multi-domain vesicle may be improved by introducing a fluid oil such as squalene, the introduction of the fluid oil enables representative poorly-soluble immunomodulatory materials insoluble in a general organic solvent to be easily solubilized, and accordingly, a multi-domain vesicle comprising the various poorly-soluble immunomodulatory materials may be produced.

## Claims

1. A multi-domain vesicle comprising: at least two liposomes making contact and connected with each other, and a multi-domain vesicle outer wall surrounding the at least two liposomes,
wherein the multi-domain vesicle is formed from an oil phase and an aqueous phase,
the oil phase comprises a first immunomodulatory material and a fluid oil, and the oil phase forms a membrane of the liposomes, and the multi-domain vesicle outer wall,
the aqueous phase comprises a second immunomodulatory material, and the aqueous phase is an internal aqueous phase of the membrane of the liposomes, and an outer aqueous phase of the membrane of the liposomes,
the first immunomodulatory material is a fat-soluble immunostimulatory material and the second immunomodulatory material is a water-soluble immunostimulatory material, and
the fluid oil improves the structural stability of the at least two liposomes making contact and connected with each other.

2. The multi-domain vesicle of claim 1, wherein the multi-domain vesicle has a size of 1 µm to 100 µm.

3. The multi-domain vesicle of claim 1, wherein the fluid oil comprises one selected from the group consisting of an animal oil, a vegetable oil, a tocopherol, mineral oil, castor oil, and combinations thereof.

4. The multi-domain vesicle of claim 3, wherein the animal oil is squalene and the vegetable oil is oleic acid.

5. The multi-domain vesicle of claim 1, wherein the fat-soluble and water-soluble immunostimulatory materials comprise a material that induces activation of antigen-presenting cells, B cells, or T cells.

6. The multi-domain vesicle of claim 1, wherein the fat-soluble immunostimulatory material comprises a material selected from the group consisting of a cationic lipid, MPLA, AGP, CRX-527, PHAD, 3D-PHAD, GLA, a lipid peptide, Pam3Cys, Pam3Cys-Lip, imiquimod (base form), resquimod (base form), VTX-2337, CRX642, a saponin (QS21), TDB, CL401, CL429, and combinations thereof.

7. The multi-domain vesicle of claim 1, wherein the water-soluble immunostimulatory material comprises a material selected from the group consisting of CpG, imiquimod (HCl form), resquimod (HCl form), Poly(I:C), STING, flagellin, a saponin, a KLK peptide, an NOD agonist peptide, Poly(dA:dT), and combinations thereof.

8. The multi-domain vesicle of claim 6, wherein the cationic lipid comprises a material selected from the group consisting of DC-cholesterol, DDA, DOTAP, DOTMA, EPC, MVL5, DODAP, and combinations thereof.

9. An immunomodulatory material comprising the multi-domain vesicle according to any one of claims 1 to 8, and an antigen.

10. The immunomodulatory material of claim 9, wherein the antigen is selected from the group consisting of a protein, a gene, a cell, a virus, and combinations thereof.

11. A method for producing a multi-domain vesicle, the method comprising steps of:
producing an oil phase solution by dissolving a first immunomodulatory material and a fluid oil in a solvent;
producing a water-in-oil (W/O) emulsion by dispersing a first aqueous phase comprising a second immunomodulatory material in the oil phase solution; and
mixing the water-in-oil emulsion with a second aqueous solution and evaporating the solvent,
wherein the first immunomodulatory material is a fat-soluble immunostimulatory material, and the second immunomodulatory material is a water-soluble immunostimulatory material.
